# EUROPEAN PATENT APPLICATION

(11) **EP 4 167 238 A1**
(43) Date of publication of application: **19.04.2023**
(21) Application number: 21202522.5
(22) Date of filing: 13.10.2021
(51) Int. Cl.: G16H 10/40, G16H 40/67

(54) **METHOD FOR OPERATING AN IN-VITRO DIAGNOSTICS LABORATORY CONTROL SOFTWARE MODULE WITH A SIMULATED IN-VITRO DIAGNOSTICS LABORATORY INSTRUMENTATION**

(71) Applicant: F. Hoffmann-La Roche AG, 4070 Basel (CH); Roche Diagnostics GmbH, 68305 Mannheim (DE)
(72) Inventor: LOETSCHER, Adrian, 6343 Rotkreuz (CH); SCHOFIELD, Christopher, 6343 Rotkreuz (CH); STACHEL, Juergen, 6343 Rotkreuz (CH)
(74) Representative: Ko, Benjamin Hyensuk

(57) **Abstract**

A method for simulating an in-vitro diagnostic IVD laboratory system (10) comprising an IVD laboratory instrumentation (2) and an IVD laboratory control software module (3) is proposed, together with a respective system (1) and various application thereof. The proposed method comprises the steps of:
- simulating sample processing within the IVD laboratory instrumentation (2) using a virtual IVD laboratory instrumentation (5) using sample processing data (SPD) provided by the IVD laboratory control software module (3),
- operating the IVD laboratory control software module (3) as if it was operating within the IVD laboratory system (10) but wherein sample processing data (SPD) intended for being exchanged between the IVD laboratory control software module (3) and the IVD laboratory instrumentation (2) is instead exchanged between the IVD laboratory control software module (3) and the virtual IVD laboratory instrumentation (5).

## Description

### TECHNICAL FIELD

The invention relates to the field of in-vitro diagnostic ("IVD") laboratory systems for processing biological samples.

### BACKGROUND

IVD testing has a major effect on medical decisions, providing physicians with pivotal information. IVD testing is in many cases performed by specialized IVD laboratory systems comprising IVD laboratory instrumentation that comprises one or more IVD laboratory instruments for processing biological samples. Within the IVD laboratory instrumentation, the samples are typically subject to complex sample processing, e.g. wherein a multitude of individual processing steps are performed on the individual sample by multiple IVD laboratory instruments. The design of an IVD laboratory instrumentation is a complicated matter that has to take into consideration a multitude of parameters and it is a complex challenge to provide a design of an IVD laboratory instrumentation that not only satisfies the desired requirements but is also able to operate efficiently.

In many IVD laboratory systems, IVD laboratory control software modules are used to control the sample processing within the IVD laboratory instrumentation, e.g. by instructing which tests are to performed when, in which order, by which IVD laboratory instrument, etc. Such IVD laboratory control software modules are highly specialized and typically require complex adaptation to the needs of the individual IVD laboratory system. The installation (including configuration, adaptation to IVD laboratory instrumentation, and/or verification) of the IVD laboratory control software modules at the site of installation of the IVD laboratory instrumentation often requires a significant amount of time, e.g. multiple months, during which the IVD laboratory instrumentation cannot (or at least cannot not fully) be used for IVD testing, which leads to commercial losses and possibly even to a shortage of IVD capacities.

### SUMMARY

It is the purpose of this invention to provide methods and systems that expand the current state of the art. For this purpose, methods and systems according to the independent claims are proposed and particular embodiments of the invention are set out in the dependent claims.

A method for simulating an in-vitro diagnostic IVD laboratory system for processing biological samples is proposed, the IVD laboratory system comprising
- an IVD laboratory instrumentation comprising one or more IVD laboratory instruments for processing samples,
- an IVD laboratory control software module for controlling sample processing within the IVD laboratory instrumentation and exchanging sample processing data between the IVD laboratory control software module and the IVD laboratory instrumentation,
wherein the IVD laboratory system is designed for
- sample processing within the IVD laboratory instrumentation using sample processing data provided by the IVD laboratory control software module;
the method comprising the steps of:
- simulating sample processing within the IVD laboratory instrumentation using a virtual IVD laboratory instrumentation using sample processing data provided by the IVD laboratory control software module,
- operating the IVD laboratory control software module as if it was operating within the IVD laboratory system but wherein sample processing data intended for being exchanged between the IVD laboratory control software module and the IVD laboratory instrumentation is instead exchanged between the IVD laboratory control software module and the virtual IVD laboratory instrumentation.

According to some embodiments, the sample processing data is indicative of test orders, processing queries, processing orders, test results, transport queries, transport orders, instrument status, workflow instructions, and/or positioning information.

According to some embodiments, the method comprises
- receiving a virtual sample and a receiving a test order for the virtual sample;
- simulating receiving of the virtual sample by the virtual IVD laboratory instrumentation resp. a virtual IVD laboratory instrument thereof;
- sending a processing query for a virtual sample from the virtual IVD laboratory instrumentation resp. a virtual IVD laboratory instrument thereof to the IVD laboratory control software module;
- sending, e.g. after having received a processing query, a processing order for a virtual sample generated using a test order for this virtual sample from the IVD laboratory control software module to the virtual IVD laboratory instrumentation resp. a virtual IVD laboratory instrument thereof;
- simulating sample processing in the form of sample testing, e.g. according to a test order the virtual IVD laboratory instrumentation resp. a virtual IVD laboratory instrument thereof, e.g. by sending a, e.g. created and/or received, test result from the virtual IVD laboratory instrumentation resp. a virtual IVD laboratory instrument thereof to the IVD laboratory control software module,
   ∘ e.g. wherein the test result is sent with a time gap from the start of the simulation of the sample testing, wherein the time gap is indicative of a time needed for this sample testing in an IVD laboratory instrumentation resp. an IVD laboratory instrument thereof;
- evaluating a received test result by the IVD laboratory control software module ;
- sending a transport query for a virtual sample from the virtual IVD laboratory instrumentation resp. a virtual IVD laboratory instrument thereof to the IVD laboratory control software module ;
- sending, e.g. after having received a transport query, a transport order for a virtual sample from the IVD laboratory control software module to the virtual IVD laboratory instrumentation resp. a virtual IVD laboratory instrument thereof;
- simulating a transport of the virtual sample within the virtual IVD laboratory instrumentation, e.g. according to a transport order, and/or
- sending positioning information from the virtual IVD laboratory instrumentation resp. a virtual IVD laboratory instrument thereof to the IVD laboratory control software module, the positioning information concerning a current simulated position of a virtual sample within the virtual IVD laboratory instrumentation resp. a virtual IVD laboratory instrument thereof.

According to some embodiments, the method comprises exchanging quality control data between the IVD laboratory control software module and the virtual IVD laboratory instrumentation.

According to some embodiments, the method comprises simulating sample processing by personnel within the IVD laboratory system.

According to some embodiments, the virtual IVD laboratory instrumentation comprises exactly one virtual IVD laboratory instrument for each IVD laboratory instrument of the IVD laboratory system to be simulated.

According to some specific embodiments, each virtual IVD laboratory instrument simulates the operational behavior of the respective IVD laboratory instrument. According to some specific embodiments, each virtual IVD laboratory instrument simulates the communicational behavior of the respective IVD laboratory instrument.

According to some embodiments, the method comprises simulating samples processed in the IVD laboratory system as virtual samples.

According to some embodiments, the method comprises displaying a visualization of the virtual IVD laboratory instrumentation together with a visualization of virtual samples.

According to some embodiments, the virtual IVD laboratory instrumentation is displayed as a schematic representation corresponding to the IVD laboratory instrumentation to be simulated.

According to some embodiments, the virtual samples each are displayed at a position in the schematic representation corresponding to a position of a corresponding sample to be simulated at the time.

According to some embodiments, the design of the virtual IVD laboratory instrumentation is created using a design file.

According to some embodiments, the design of the virtual IVD laboratory instrumentation is created using a list of IVD laboratory instruments defined in the IVD laboratory control software module.

According to some embodiments, the design of the virtual IVD laboratory instrumentation are created using a graphical user interface.

According to some specific embodiments, the method comprises using a graphical user interface that comprises a schematic representation of a visualization of details of the design of the virtual IVD laboratory instrumentation.

According to some embodiments,
- simulating sample processing within the IVD laboratory instrumentation and
- operating the IVD laboratory control software module,
are both performed in real-time.

According to some embodiments, the method comprises
- simulating sample processing within the IVD laboratory instrumentation in a simulation time;
- operating the IVD laboratory control software module in real-time;
- comparing the time passed in the simulation time to the real-time;
- and:
   ∘ increasing the speed of the simulation time if the simulation time is behind the real-time,
   ∘ decreasing the speed of the simulation time if the simulation time is ahead of the real-time, or
   ∘ maintaining the speed of the simulation time if the simulation time is at least quasi-identical to the real-time.

According to some embodiments, the IVD laboratory control software module is connected to a data management system for exchanging sample related information with the IVD laboratory control software module, and
wherein the method comprises
- exchanging sample related information between the IVD laboratory control software module and the data management system.

According to some embodiments, the method comprises
- simulating a virtual data management system configured for exchanging sample related information with the IVD laboratory control software module, and
- exchanging sample related information between the IVD laboratory control software module and the virtual data management system.

According to some specific embodiments, the method comprises
- receiving by the IVD laboratory control software module a first data from the virtual data management system or from the virtual IVD laboratory instrumentation, the first data comprising an indication that a sample has arrived at the IVD laboratory instrumentation, thereby receiving a virtual sample;
- receiving by the IVD laboratory control software module a second data from the virtual data management system, the second data comprising a test order for the sample, thereby receiving a test order for the virtual sample;
- simulating sample processing of this virtual sample according to the test order using the virtual IVD laboratory instrumentation.

A method for designing an IVD laboratory control software module using one of the proposed method for simulating is proposed, the method for designing comprising the following steps in the following order:
- STEP 0: providing a design of the IVD laboratory control software module ;
- STEP 1: performing the method for simulating;
- STEP 2: changing one or more parameters of the design of the IVD laboratory control software module ;
- STEP 3: performing STEP 1; and
- STEP 4: optionally repeating STEP 2 and STEP 3.

A method for designing an IVD laboratory instrumentation using one of the proposed method for simulating is proposed, the method for designing comprising the following steps in the following order:
- STEP 0: providing a design of the IVD laboratory instrumentation ;
- STEP 1: performing the method for simulating;
- STEP 2: changing one or more parameter of the design of the IVD laboratory instrumentation ;
- STEP 3: performing STEP 1; and
- STEP 4: optionally repeating STEP 2 and STEP 3.

A method for designing an IVD laboratory system using one of the proposed method for simulating is proposed, the method for designing comprising the following steps in the following order:
- STEP 0: providing a design of the IVD laboratory control software module and a design of the IVD laboratory instrumentation ;
- STEP 1: performing the method for simulating;
- STEP 2: changing one or more parameter of the design of the IVD laboratory control software module and/or of the design of the IVD laboratory instrumentation ;
- STEP 3: performing STEP 1; and
- STEP 4: optionally repeating STEP 2 and STEP 3.

According to some embodiments, STEP 1 of the proposed methods having a STEP 1 may comprise benchmarking the performance of the IVD laboratory control software module resp. the IVD laboratory instrumentation.

A method for testing an IVD laboratory control software module using one of the proposed method for simulating is proposed.

A method for testing an IVD laboratory instrumentation using one of the proposed method for simulating is proposed.

A method for testing an IVD laboratory system using one of the proposed method for simulating is proposed.

A method for testing personnel operation of an IVD laboratory system using one of the proposed method for simulating is proposed.

A method for training personnel for working in an IVD laboratory system using one of the proposed method for simulating is proposed.

A method for designing a virtual IVD laboratory instrument for simulating an IVD laboratory instrument for one of the proposed method for simulating is proposed, the method comprising
- recording and/or defining data files being received and/or being sent by the IVD laboratory instrument, and/or
- recording and/or defining time gaps between the receiving and/or the sending of the data files by the IVD laboratory instrument ;
- using the recorded resp. defined data files and/or the recorded resp. defined time gaps for creating the content and/or for defining the sending timing of data files by the virtual IVD laboratory instrument.

A system for simulating an IVD laboratory system for processing biological samples is proposed, the IVD laboratory system comprising
- an IVD laboratory instrumentation comprising one or more IVD laboratory instruments for processing biological samples and
- an IVD laboratory control software module for controlling sample processing within the IVD laboratory instrumentation and exchanging sample processing data between the IVD laboratory control software module and the IVD laboratory instrumentation,
wherein the IVD laboratory system is designed for
- sample processing within the IVD laboratory instrumentation using sample processing data provided by the IVD laboratory control software module ;
wherein the system comprises
- the IVD laboratory control software module, and
- a simulation software module comprising a virtual IVD laboratory instrumentation for simulating sample processing within the IVD laboratory instrumentation ;
wherein the system is configured for
- operating the IVD laboratory control software module as if it was operating within the IVD laboratory system, but wherein sample processing data intended for being exchanged between the IVD laboratory control software module and the IVD laboratory instrumentation is instead exchanged between the IVD laboratory control software module and the virtual IVD laboratory instrumentation ;
wherein the virtual IVD laboratory instrumentation is configured for
- simulating sample processing within the IVD laboratory instrumentation using sample processing data provided by the IVD laboratory control software module.

According to some embodiments, the IVD laboratory control software module comprises a first communication module comprising at least one driver for transforming a data format of a data file.

According to some embodiments, the simulation software module comprises a second communication module comprising at least one driver for transforming a data format of a data file.

According to some embodiments, the first communication module and the second communication module each comprise at least one joint driver.

According to some embodiments, the operation of the first communication modules is mirrored by the second communication module.

### BRIEF DESCRIPTION OF THE DRAWINGS

Specific embodiments of the invention are described in the following with reference to the drawings, which are for the purpose of illustrating the present specific embodiments of the inventions and not for limiting the same. In the drawings,
- Fig. 1: shows an IVD laboratory system,
- Fig. 2: shows a scheme of operation of an IVD laboratory system,
- Fig. 3: shows a scheme of operation of an IVD laboratory system,
- Fig. 4: shows a scheme of operation of a simulation of an IVD laboratory system,
- Fig. 5: shows a scheme of operation of a simulation of an IVD laboratory system,
- Fig. 6: shows a scheme of operation of a simulation of an IVD laboratory system,
- Fig. 7: shows a scheme of operation of a simulation of an IVD laboratory system,
- Fig. 8: shows a scheme of operation of a simulation of an IVD laboratory system,
- Fig. 9: shows a scheme of operation of a simulation of an IVD laboratory system,
- Fig. 10: shows a scheme of operation of a simulation of an IVD laboratory system,
- Fig. 11: shows a scheme of operation of a simulation of an IVD laboratory system,
- Fig. 12: shows a scheme of exchanging sample processing data in an IVD laboratory system,
- Fig. 13: shows a scheme of exchanging sample processing data in a simulation of an IVD laboratory system,
- Figs. 14a,b: each show a visualization of a simulated IVD laboratory, and
- Fig. 15: shows possible use cases for the proposed methods/systems.

### DESCRIPTION OF SPECIFIC EMBODIMENTS

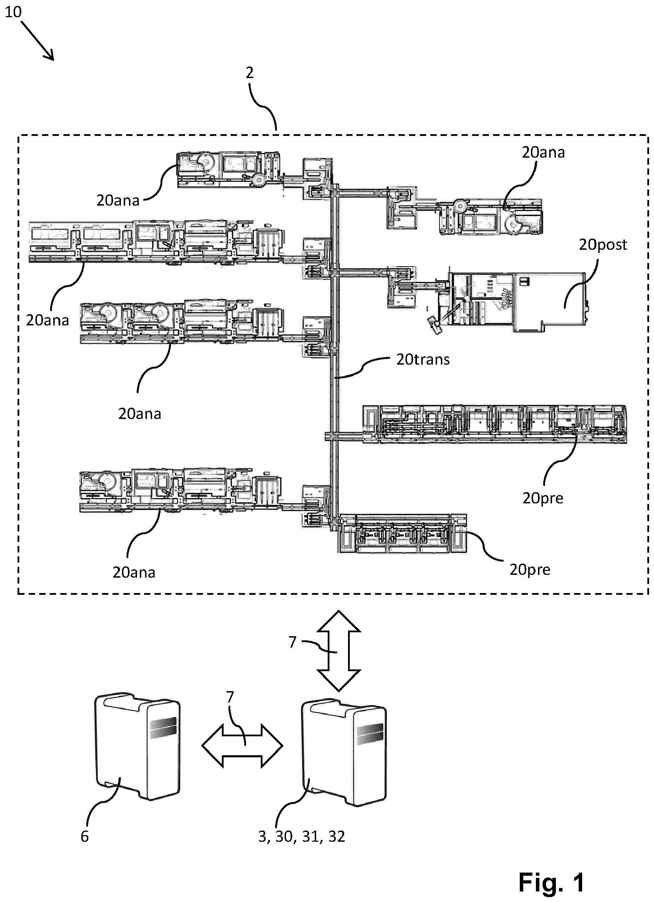

**Fig. 1** illustrates an example of an in-vitro diagnostic, herein abbreviated as "IVD", laboratory system 10 for processing biological samples.

A biological sample can comprise a biological material, e.g. as taken from a human body or an animal body. A biological sample can comprise a body fluid, such as blood, interstitial fluid, urine, saliva, or other types of body fluids. For simplicity, biologicals samples are herein typically only referred to as "samples".

A sample can potentially comprise at least one analyte of interest, e.g. molecules, ions, proteins, metabolites, pathogens, and the like. It is typically one of the tasks of IVD testing to detect the presence resp. absence and/or a concentration of one or more analytes in a sample. More generically, IVD testing can refer to determining a biological property of a sample. IVD testing can comprise performing at least one analytical test on a sample, wherein the analytical test can allow to draw conclusions on the biological properties of the sample. The analytical test can e.g. comprise adding a reagent to the sample, a possible detectable reaction of the sample with the reagent, and/or a detecting or non-detection of this reaction. Detecting of an reaction can e.g. comprise measuring a physical value of the sample (resp. a composite obtained by using the sample such as a sample-reagent mixture), such as a spectrum and/or an intensity of a radiation reflected by and/or transmitted through the sample (resp. the composite obtained by using the sample).

Processing a sample can e.g. comprise transporting the sample (typically in IVD containers such as IVD tubes; the IVD containers may be held in IVD container holders such as IVD tube racks), performing pre-analytical steps on the samples (e.g. preparatory steps such as centrifuging), performing analytical steps on the samples (e.g. adding a reagent to the sample and measuring the reaction of the sample with the reagent), and/or performing post-analytical steps on the samples (e.g. storing of a sample in a refrigerator for later use).

The IVD laboratory instrumentation 2 comprises one or more IVD laboratory instruments 20 designed for processing samples, e.g. for performing one or more steps of an intended workflow on the sample. Processing a sample can comprise one or more physical processing steps (e.g. moving, mixing, heating, etc.). An IVD laboratory instruments 20 can comprise instrument hardware for processing samples (e.g. gripper, reagent storage, pipetting apparatus, heating element, etc.) as well as instrument software designed for operating the instrument hardware. An IVD laboratory instrument 20 can comprise a control unit designed for controlling, in particular steering, the operation of the instrument hardware, wherein the instrument software can be designed for being executed using the control unit.

The IVD laboratory instruments 20 are typically categorized according to the different type of sample processing steps they can perform. A transport IVD laboratory instrument 20trans is designed for transporting samples (resp. the IVD containers and/or respective holders), e.g. from one IVD laboratory instrument to another. A pre-analytical IVD laboratory instruments 20pre is designed for performing pre-analytical steps on the samples. A analytical IVD laboratory instrument 20ana is designed for performing analytical steps (such as an analytical test) on the samples; an analytical IVD laboratory instrument 20ana can comprise a digital analytical IVD laboratory instrument designed for performing analytical computation steps (e.g. a medical algorithm). A post-analytical IVD laboratory instrument 20post is designed for performing post-analytical steps on the samples. Some IVD laboratory instruments 20 are capable of performing multiple type of sample processing steps, e.g. pre-analytical and analytical steps. In the example of Fig. 1, the IVD laboratory instrumentation 2 comprises two pre-analytical IVD laboratory instruments 20pre, five analytical IVD laboratory instruments 20ana, one post-analytical IVD laboratory instruments 20post, and one transport IVD laboratory instruments 20trans, wherein the IVD laboratory instrumentation 2 illustrated in Fig. 1 is designed such that the one transport IVD laboratory instruments 20trans connects all the other IVD laboratory instruments 20.

An IVD laboratory system 10 can further comprise an IVD laboratory control software module 3 designed for controlling sample processing within the IVD laboratory instrumentation 2, e.g. wherein
- the IVD laboratory control software module 3 is designed for exchanging sample processing data SPD between the IVD laboratory control software module 3 and the IVD laboratory instrumentation 2 and
- the IVD laboratory system 10 is designed for sample processing within the IVD laboratory instrumentation 2 using sample processing data SPD provided by the IVD laboratory control software module 3.

Controlling sample processing can comprise steering the sample processing (e.g. instructing workflow(s) resp. workflow step(s) for the sample processing) and/monitoring the sample processing (e.g. receiving and processing data concerning the sample processing). The monitoring can influence the steering (e.g. where the IVD laboratory control software module 3 deems that an analytical test is invalid, the IVD laboratory control software module 3 can initiate that the test is rerun).

Controlling sample processing within the IVD laboratory instrumentation 2 can e.g. comprise instructing the IVD laboratory instrumentation 2, managing the status of the IVD laboratory instrumentation 2, tracking the (current) positions of the samples, tracking the processing step(s) (currently) applied to the samples, receiving and possibly validating test results. sample processing data SPD can be used for controlling the sample processing within the IVD laboratory instrumentation 2 e.g. in that the communication between the IVD laboratory control software module 3 and the IVD laboratory instrumentation 2 is realized by using sample processing data SPD and/or in that the sample processing data SPD is used for determining parameters used for the coordination, e.g. a status and or a value used for validation.

According to some embodiments, the IVD laboratory control software module 3 is designed for making decisions in the context of sample processing, e.g. which processing step is to be taken by which IVD laboratory instrument 20 and/or if a test result is deemed valid.

According to some embodiments, the IVD laboratory control software module 3 is designed for steering the sample processing within the IVD laboratory instrumentation 2 by sending, to the IVD laboratory instrumentation 2, respective sample processing data SPD, which can e.g. be indicative of a workflow for a sample and/or of which IVD laboratory instrument 20 shall conduct which processing step for a sample.

According to some embodiments, the IVD laboratory control software module 3 is designed for monitoring the sample processing within the IVD laboratory instrumentation 2 by receiving, from the IVD laboratory instrumentation 2, respective sample processing data SPD, which can e.g. be indicative of a position of a certain sample within the IVD laboratory instrumentation 2 and/or be indicative of a result of an analytical test by an analytical IVD laboratory instrument 20ana.

According to some embodiments, the IVD laboratory control software module 3 is designed for controlling the sample processing as in between the IVD laboratory instruments 20, e.g. that a specific analytical IVD laboratory instrument 20ana will perform a specific analytical test, and each IVD laboratory instrument 20 is designed for controlling the details of the sample processing within the IVD laboratory instrument 20 itself, e.g. the individual workflow steps of the analytical tests within an analytical IVD laboratory instrument 20ana are managed by said analytical IVD laboratory instrument 20ana itself.

According to some embodiments, the IVD laboratory control software module 3 is designed for controlling the processing of one or more samples based on test orders for the samples. The test order can be received by the IVD laboratory control software module 3, e.g. from a data management unit 6 or via an input using a user interface. The IVD laboratory control software module 3 can comprise a communication module which can e.g. be designed for receiving the test orders corresponding to the samples. A test orders can be indicative of processing steps (such as analytical measurements) to be carried out on the respective sample.

According to some embodiments, the IVD laboratory system 10 can comprise a data management unit 6 such as a Laboratory Information System ("LIS") or a Hospital Information System ("HIS"). The data management unit 6 can be designed for inputting, storing, organizing, and/or accessing data, e.g. sample related information SRI such test orders, test results, and/or patient data. The data management unit 6 can comprise one or more interfaces for interacting with data consumers, data creators, and/or an IVD laboratory control software module 3. According to some embodiments, the data management unit 6 is intended for being accessed by the personnel of a hospital and/or laboratory and used for managing the medically relevant data of a hospital resp. laboratory. The data management unit 6 can be connected (e.g. via a communication network 7 as depicted in the example of Fig. 1) to the IVD laboratory control software module 3, e.g. for exchanging sample related information SRI. The data management unit 6 can be designed for sending test orders and/or personal data relating to samples to be processed to the IVD laboratory control software module 3. The IVD laboratory control software module 3 can be designed for sending test results relating to test orders to the data management unit 6.

According to some embodiments, the (e.g. hospital and/or laboratory) personnel (e.g. nurses, physicians etc.) can use an data management unit 6 for managing medically relevant data including sample related information SRI. The personnel can enter sample related information SRI such as test orders into the data management unit 6; the data management unit 6 can be connected to the IVD laboratory control software module 3 and can send sample related information SRI such as test orders to the IVD laboratory control software module 3; the IVD laboratory control software module 3 can steer the IVD laboratory instrumentation 2 to conduct test orders by using sample processing data SPD (some which can e.g. be created using the sample related information SRI); the IVD laboratory control software module 3 can receive test results relating to test order from the IVD laboratory instrumentation 2; the IVD laboratory control software module 3 can send sample related information SRI such as test results to the data management unit 6, and/or the personnel can access test results via the data management unit 6.

According to some embodiments, the IVD laboratory control software module 3 is designed for serving as an intermediate between a data management unit 6, such as a HIS and/or a LIS, on the one side and an IVD laboratory instrumentation 2 on the other side; in this case the IVD laboratory control software module 3 is sometimes also referred to as IVD laboratory middleware.

According to some embodiments, the IVD laboratory control software module 3 is designed for being executed by a control unit, e.g. a control unit of the IVD laboratory system 10 (e.g. a control unit of an individual IVD laboratory instrument 20 thereof). The control unit can e.g. be implemented using a PC, a server, and/or a cloud-solution. The example of Fig. 1 depicts the IVD laboratory control software module 3 being comprised in a control unit 32 of the IVD laboratory system 10.

According to some embodiments, the IVD laboratory control software module 3 comprises one or more computer programs designed for being executed by one or more processing units (e.g. of a control unit).

According to some embodiments, the IVD laboratory control software module 3 can be embodied as a programmable logic controller running a computer-readable program provided with instructions to perform operations.

An IVD laboratory control software module 3 typically is a very specialized and complex software product, such as the software *cobas*^{®} *infinity laboratory solution* of *Roche Diagnostics* as available on 01^{th} of October 2021.

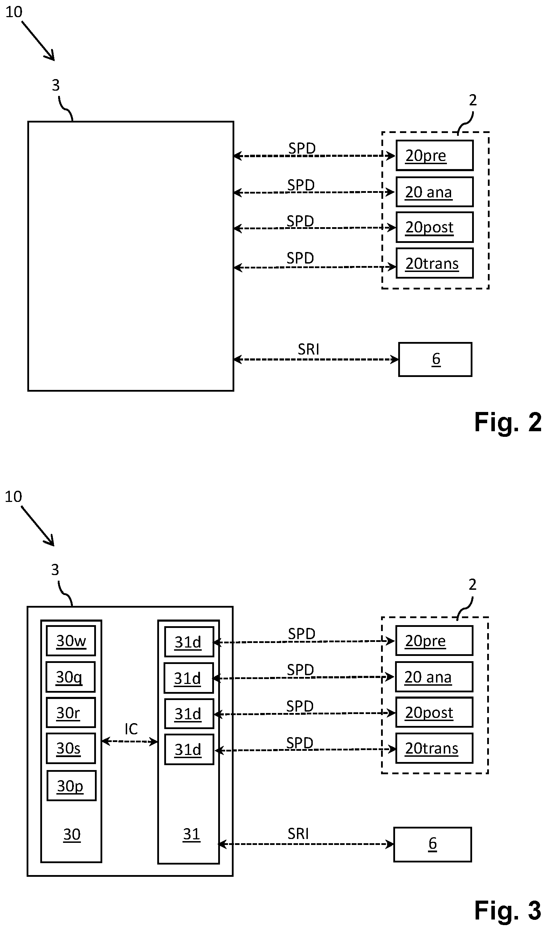

**Fig. 2** shows a possible schema of operating an IVD laboratory system 10. The IVD laboratory control software module 3 is in communication with the IVD laboratory instrumentation 2 by exchanging sample processing data SPD and thereby controls the sample processing within the IVD laboratory instrumentation 2. The IVD laboratory instrumentation 2 can comprise multiple IVD laboratory instruments 20 of various types for performing the various steps of sample processing, e.g. based on sample processing data SPD provided by the IVD laboratory control software module 3. The IVD laboratory control software module 3 can e.g. provide the IVD laboratory instrumentation 2 with sample processing data SPD indicating in which order which sample processing steps shall be performed on which samples, and the IVD laboratory instrumentation 2 can perform the sample processing steps accordingly. The IVD laboratory instrumentation 2 can e.g. provide the IVD laboratory control software module 3 with sample processing data SPD indicating queries, status reports, and test results, which are created by the IVD laboratory instrumentation 2 (e.g. by the individual IVD laboratory instruments 20 thereof). The IVD laboratory control software module 3 can e.g. react to a received query by sending sample processing data SPD indicating an instruction to the IVD laboratory instrumentation 2; to a received status report by updating a dashboard indicating the status of the IVD laboratory instrumentation 2; to a received test result by managing test result (e.g. validating the test results).

Sample processing data SPD refers to data concerning the processing of the sample within the IVD laboratory system 10. The IVD laboratory control software module 3 can use sample processing data SPD for controlling the sample processing within the IVD laboratory instrumentation 2. Sample processing data SPD can e.g. comprise test orders, processing queries, processing orders, test results, transport queries, transport orders, instrument status, workflow instructions, and/or positioning information. Sample processing data SPD can e.g. comprise workflow instructions from the IVD laboratory control software module 3 to the IVD laboratory instrumentation 2 as to how a sample is to be processed (e.g. which analytical tests are to be performed by which IVD laboratory instrument 20). Sample processing data SPD can e.g. comprise query or information sent from the IVD laboratory instrumentation 2 to the IVD laboratory control software module 3 (e.g. a processing query as to what to do next with the sample or information such as a test result of an analytical test). According to an example, after receiving a sample and a respective test order in the IVD laboratory system 10, the IVD laboratory control software module 3 plans the processing of this sample according to the test order and sends sample processing data SPD to the IVD laboratory instrumentation 2 that instruct the IVD laboratory instrumentation 2 on to how to process the sample and the IVD laboratory instrumentation 2 processes the sample based on these instructions; during the processing of the sample, the IVD laboratory instrumentation 2 sends sample processing data SPD comprising status reports to the IVD laboratory control software module 3 so that the IVD laboratory control software module 3 can monitor the status of the processing of the sample; after a measurement step performed by the IVD laboratory instrumentation 2, the IVD laboratory instrumentation 2 sends sample processing data SPD comprising measurement results (e.g. results of an analytical test) to the IVD laboratory control software module 3 so that the IVD laboratory control software module 3 can administrate the measurement results. Sample processing data SPD can e.g. be realized in a data structure in compliance with the Health Level Seven standard (also referred to as "HL7") or an ASTM standard; both as e.g. available on 01^{th} of October 2021).

According to some embodiments, controlling the sample processing within the IVD laboratory instrumentation 2 comprises instructing as to how to perform the sample processing within the IVD laboratory instrumentation 2.

According to some embodiments, controlling the sample processing within the IVD laboratory instrumentation 2 comprises monitoring the sample processing within the IVD laboratory instrumentation 2.

According to some embodiments, exchanging sample processing data SPD between the IVD laboratory control software module 3 and the IVD laboratory instrumentation 2 comprises transmitting sample processing data SPD from the IVD laboratory control software module 3 to the IVD laboratory instrumentation 2 (e.g. to a particular IVD laboratory instrument 20 of the IVD laboratory instrumentation 2), e.g. test orders, processing orders, transport orders, and/or workflow instructions.

According to some embodiments, exchanging sample processing data SPD between the IVD laboratory control software module 3 and the IVD laboratory instrumentation 2 comprises transmitting sample processing data SPD from the IVD laboratory instrumentation 2 (resp. a particular IVD laboratory instrument 20 of the IVD laboratory instrumentation 2) to the IVD laboratory control software module 3, e.g. processing queries, test results, transport queries, instrument status, and/or positioning information.

According to some embodiments, the IVD laboratory control software module 3 comprises a workflow engine 30w designed for planning the sample processing within the IVD laboratory instrumentation 2 to which the IVD laboratory control software module 3 is connected. According to some specific embodiments, the workflow engine 30w has knowledge of the capabilities and capacities of each IVD laboratory instrument 20 of the IVD laboratory instrumentation 2 and is designed for planning which sample processing step is to be taken by which IVD laboratory instrument 20. The workflow engine 30w can be designed for planning in order to achieve a balancing the load in between the IVD laboratory instrument 20. The workflow engine 30w can be designed for planning taking into consideration a priority allocated to samples, e.g. wherein the priority is allocated using the test order of a respective sample (e.g. a priority value that is comprised in the test order and that e.g. was specified by a physician). The workflow engine 30w can be designed for planning an efficient route through the IVD laboratory instrumentation 2 to avoid unbeneficial movement of the sample, which can e.g. allow lowering the risk of cross-contamination. The workflow engine 30w can be designed for planning taking into consideration aliquoting, which can e.g. allow for parallel testing. The workflow engine 30w can be designed for planning taking into consideration parallel testing, e.g. wherein two or more parts of the sample are processed in parallel, e.g. in two or more different IVD laboratory instruments 20; this can allow to lower the time needed to produce test results. The workflow engine 30w can be designed for planning masking resp. unmasking of IVD laboratory instruments 20, which can e.g. allow reducing reagent waste. The workflow engine 30w can be designed for re-planning, e.g. upon certain events such as when a new test order is received, when a rerun of a test is to be conducted (e.g. because an error had occurred during the first test), when reflex test is to be conducted (e.g. a follow up test that is to be conducted because of the result of an earlier test), and/or when an IVD laboratory instrument 20 is masked or unmasked. According to some specific embodiments, the IVD laboratory control software module 3 uses the planning of the workflow engine 30w for controlling, in particular for steering, of the sample processing within the IVD laboratory instrumentation 2, e.g. by sending, to the IVD laboratory instrumentation 2, sample processing data SPD comprising instructions that, when executed by the IVD laboratory instrumentation 2, will cause the IVD laboratory instrumentation 2 to process the samples according to the planning. The IVD laboratory control software module 3 can comprise an interface for specifying algorithms, parameters etc. of the workflow engine 30w. The workflow engine 30w can comprise an optimization module for optimizing the planning of sample processing within the IVD laboratory instrumentation 2; the optimization module can comprise an artificial intelligence unit.

According to some embodiments, the IVD laboratory control software module 3 comprises a quality control ("QC") module 30q designed for managing QC data provided by the IVD laboratory instrumentation 2. The QC data can comprise results of QC measurements conducted by IVD laboratory instruments 20 of the IVD laboratory instrumentation 2; sample processing data SPD sent from the IVD laboratory instrumentation 2 to the IVD laboratory control software module 3 can be indicative of QC measurements results.

According to some embodiments, the IVD laboratory control software module 3 comprises a rule engine 30r designed for validating a test result. The validation can be a technical and/or a medical validation; a technical validation e.g. being that the QC of the IVD laboratory instrumentation 2 on which the test result was produced is accepted; a medical validation e.g. being that the test result is within an acceptable range; the acceptable range can e.g. depend on respective patient data and/or other test results for the same sample. The IVD laboratory control software module 3 can comprise an interface for specifying algorithms, parameters etc. of the rule engine 30r.

According to some embodiments, the IVD laboratory control software module 3 comprises a sample tracking module 30s designed for tracking the position of samples within the IVD laboratory instrumentation 2. The sample tracking module 30s can e.g. be designed for tracking the position using sample processing data SPD being indicative of position data (as e.g. sent from the IVD laboratory instrumentation 2 to the IVD laboratory control software module 3).

According to some embodiments, the IVD laboratory control software module 3 comprises a patient data module 30p for managing patient data of patients whose samples are being processed. The patient data can e.g. be provided by a data management unit 6 and/or inputted by a user interface. The patient data can e.g. be used for validating - by the IVD laboratory control software module 3 - test results and/or for ordering - by the IVD laboratory control software module 3 - additional tests in reaction to certain test results. The patient data module 30p can. comprise a database for storing patient data, e.g. name, identifier, age, medical history, etc.

**Fig. 3** shows a possible schema of operating an IVD laboratory system 10 similar to that of Fig. 2, but in addition shows details on the control components 30 of the IVD laboratory control software module 3; in the illustrated example, the control components 30 comprise a workflow engine 30w, a rule engine 30r, a QC module 30q, a sample tracking module 30s, and a patient data module 30p.

According to some embodiments, the IVD laboratory control software module 3 comprises an artificial intelligence unit, e.g. designed for being used in the context of the workflow engine and/or the rule engine. An artificial intelligence unit can e.g. be comprised in one or more of the control components 30.

According to some embodiments, the IVD laboratory control software module 3 comprises a communication module 31 comprising at least one driver 31d for transforming a data format of a data file, e.g. for converting a data file, e.g. a data file comprising sample processing data SPD, from first data format to a second data format. This can e.g. allow using the IVD laboratory control software module 3 with IVD laboratory instruments 20 that use other data formats than the IVD laboratory control software module 3. In an example, sample processing data SPD is created by the control components 30 in a data format not processible by the IVD laboratory instrumentation 2, and the communication module is designed for converting sample processing data SPD to a data format that is processible by the IVD laboratory instrumentation 2 - and/or vice versa. It can e.g. be the case that at least some of the IVD laboratory instruments 20 of the IVD laboratory instrumentation 2 use the HL7 data format standard, while the control components 30 use a proprietary data format that per se is not compatible with the HL7 standard; in this case the communication module 31 can allow converting the files from HL7 to the proprietary data format - and vice versa. According to some specific embodiments, the communication module comprises a driver 30d for each IVD laboratory instrument 20 of the IVD laboratory instrumentation 2. The communication module 31 can be in communication with the control components 30 of the IVD laboratory control software module 3 for internal communication IC within the IVD laboratory control software module 3. An example of an IVD laboratory control software module 3 comprising a (first) communication module 31 is illustrated in Fig. 3.

According to some embodiments, a data management unit 6, such as a LIS or a HIS) designed for administration within the laboratory resp. the hospital, is connected to the IVD laboratory control software module 3 for exchanging sample related information SRI. As shown in the example of Fig. 3, the data management unit 6 can e.g. be connected to the IVD laboratory control software module 3 via a (first) communication module 31 of the IVD laboratory control software module 3.

A method for simulating an IVD laboratory system 10 for processing biological samples is proposed, wherein the IVD laboratory system 10 to be simulated comprises
- an IVD laboratory instrumentation 2 comprising one or more IVD laboratory instruments 20 for processing samples,
- an IVD laboratory control software module 3 for controlling sample processing within the IVD laboratory instrumentation 2 and exchanging sample processing data SPD between the IVD laboratory control software module 3 and the IVD laboratory instrumentation 2,
and wherein the IVD laboratory system 10 to be simulated is designed for
- sample processing within the IVD laboratory instrumentation 2 using sample processing data SPD provided by the IVD laboratory control software module 3.

The proposed method comprises the steps of:
- simulating sample processing within the IVD laboratory instrumentation 2 using a virtual IVD laboratory instrumentation 5 using sample processing data SPD provided by the IVD laboratory control software module 3,
- operating the IVD laboratory control software module 3 as if it was operating within the IVD laboratory system 10 but wherein sample processing data SPD intended for being exchanged between the IVD laboratory control software module 3 and the IVD laboratory instrumentation 2 is instead exchanged between the IVD laboratory control software module 3 and the virtual IVD laboratory instrumentation 5.

The proposed method can allow operating the IVD laboratory control software module 3 as if it was interacting with a real-world IVD laboratory instrumentation 2, but wherein the sample processing data SPD is instead exchanged with a virtual IVD laboratory instrumentation 5 and the sample processing is only simulated. The virtual IVD laboratory instrumentation 5 can e.g. be designed for representing the IVD laboratory instrumentation 2 and provide the same kind of sample processing data SPD to the IVD laboratory control software module 3 as the IVD laboratory instrumentation 2 in the real-world would. The proposed method can allow for simulating a sample flow as well as a respective data flow within the IVD laboratory system 10. The proposed method can allow for testing the IVD laboratory control software module 3 and/or an IVD laboratory design resembled (e.g. embodied) by the virtual IVD laboratory instrumentation 5.

The virtual IVD laboratory instrumentation 5 can be designed to react - from the perspective of the IVD laboratory control software module 3 - to the sample processing data SPD in essentially the same way as a real-world IVD laboratory instrumentation 2 would. According to some embodiments, the virtual IVD laboratory instrumentation 5 is designed for sending to the IVD laboratory control software module 3 - e.g. in reaction to receiving a specific type of sample processing data SPD from the IVD laboratory control software module 3 - the same type of sample processing data SPD in the same manner as an IVD laboratory instrumentation 2 would in reaction to receiving that specific type of sample processing data SPD from the IVD laboratory control software module 3. According to an example, the virtual IVD laboratory instrumentation 5 receives the instruction to conduct a certain analytical test, in reaction to which the IVD laboratory control software module 3 in real-world operation would receive back a respective test result from the IVD laboratory instrumentation 2 in HL7 format; and later, the virtual IVD laboratory instrumentation 5 sends to the IVD laboratory control software module 3 a generated (e.g. randomly generated according to a pre-determined distribution) test result in HL7 format; wherein the time difference between the reception of the instruction and the sending of the test result by the virtual IVD laboratory instrumentation 5 can e.g. resemble the time needed for providing the test result if the analytical test would have been conducted in the real-world IVD laboratory instrumentation 2; the time difference can e.g. depend on the number and/kind of tests currently being simulated to be processed by the virtual IVD laboratory instrumentation 5 so as to resemble the waiting time a similar workload would cause in the real-world. This can allow running the IVD laboratory control software module 3 essentially in the same way as if it was running in the real-world IVD laboratory system 10, which can e.g. allow testing the IVD laboratory control software module 3 such as to stress-test it and/or to test its behavior under certain conditions.

According to some embodiments, simulating sample processing within the IVD laboratory instrumentation 2 using a virtual IVD laboratory instrumentation 5 using sample processing data SPD provided by the IVD laboratory control software module 3 comprises that the virtual IVD laboratory instrumentation 5 receives sample processing data SPD from the IVD laboratory control software module 3 and in reaction to the received sample processing data SPD - and possibly with a time delay - sends respective sample processing data SPD back to IVD laboratory control software module 3.

According to some embodiments, the IVD laboratory control software module 3 is operated in the proposed method as if it was operating within the real-world in that the IVD laboratory control software module 3 is agnostic of the fact that it runs in a simulation context. According to an example, no configuration parameter of the IVD laboratory control software module 3 is specific to a simulation context as such, e.g. such that the IVD laboratory control software module 3 could operate with a (suitable) real-world IVD laboratory instrumentation 2 using the same configuration.

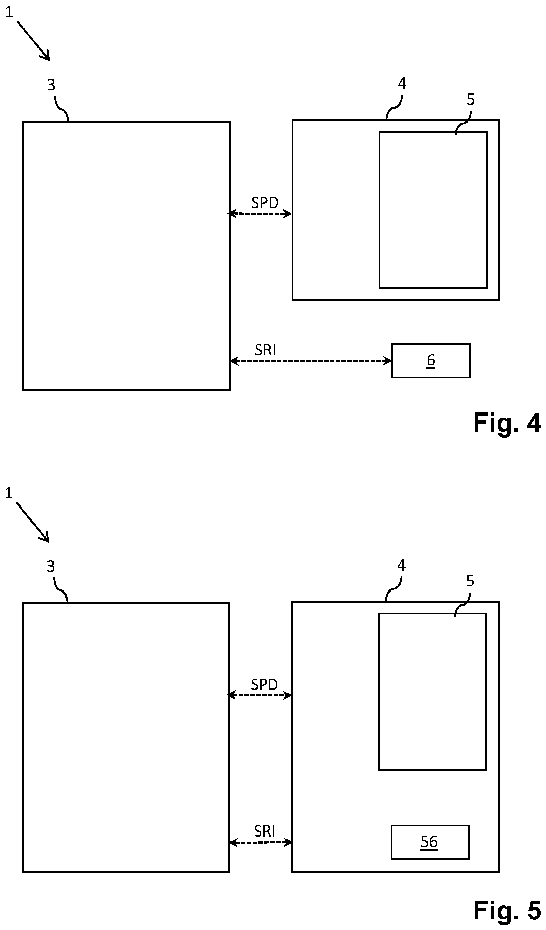

The proposed method can e.g. be realized using one of the systems 1 schematically exemplified in one of Figs. 4 to 11.

**Fig. 4** shows the possible schema of performing the proposed method: The IVD laboratory control software module 3 is connected to a simulation software module 4 that is used to simulate the operation of the IVD laboratory instrumentation 2 by operating a respective virtual IVD laboratory instrumentation 5. The IVD laboratory control software module 3 is in communication with the virtual IVD laboratory instrumentation 5 by exchanging sample processing data SPD, e.g. in an HL7 or ASTM format, and thereby controls the simulation of the sample processing within the IVD laboratory instrumentation 2 using the virtual IVD laboratory instrumentation 5. The IVD laboratory control software module 3 can e.g. provide the virtual IVD laboratory instrumentation 5 with sample processing data SPD indicating in which order which sample processing steps shall be performed, and the virtual IVD laboratory instrumentation 5 can simulate the sample processing steps accordingly. The virtual IVD laboratory instrumentation 5 can e.g. provide the IVD laboratory control software module 3 with sample processing data SPD indicating queries, status reports, and/or test results, which are e.g. created by virtual IVD laboratory instrumentation 5 (e.g. according to certain pre-determined rules) or which are e.g. copies of sample processing data SPD earlier created by real-world IVD laboratory instruments 20. In analogy to the real-world situation, the IVD laboratory control software module 3 can e.g. react to a received query by sending of sample processing data SPD indicating an instruction to the virtual IVD laboratory instrumentation 5; to a received status report by updating a dashboard indicating the status of the alleged IVD laboratory instrumentation 2; to a received test result by managing test result (e.g. validate test results). The IVD laboratory control software module 3 can act in essentially the same way as if it was interacting with a real-world IVD laboratory instrumentation 2, although it de facto interacts with a simulation thereof in form of a virtual IVD laboratory instrumentation 5. The virtual IVD laboratory instrumentation 5 can be designed for reacting to sample processing data SPD provided by the IVD laboratory control software module 3 similarly (e.g. essentially in the same way) as an IVD laboratory instrumentation 2 would react to the same sample processing data SPD, e.g. by replying to the IVD laboratory control software module 3 with sample processing data SPD of the same kind, format, and/or content as a real-world IVD laboratory instrumentation 2 would. The virtual IVD laboratory instrumentation 5 can be designed to resemble an existing IVD laboratory instrumentation 2; this can e.g. allow to test the IVD laboratory control software module's performance with the existing IVD laboratory instrumentation 2. The virtual IVD laboratory instrumentation 5 can be designed according to a planned design for an IVD laboratory instrumentation 2; that can e.g. allow to test the performance of the design without actually creating the IVD laboratory instrumentation 2 in the real-world.

According to some embodiments, the virtual IVD laboratory instrumentation 5 simulates the sample flow within the IVD laboratory instrumentation 2 according to the sample processing data SPD provided by the IVD laboratory control software module 3 to the virtual IVD laboratory instrumentation 5; and the data flow between the IVD laboratory control software module 3 and the virtual IVD laboratory instrumentation 5 can be similar (e.g. identical) to the data flow in a real world IVD laboratory system 10, e.g. in that the IVD laboratory control software module 3 sends and receives the same type of sample processing data SPD as if it was operating in a respective IVD laboratory system 10.

The proposed method can be realized as a computer-implemented method. According to some embodiments, the method comprises running an IVD laboratory control software module 3 and a virtual machine on the same and/or different computation entities (e.g. computers, cloud environments), wherein the virtual IVD laboratory instrumentation 5 is simulated using the virtual machine and data, in particular sample processing data SPD, is exchanged between the IVD laboratory control software module 3 and the virtual machine.

According to some embodiments, the IVD laboratory control software module 3 is connected to a data management system 6 designed for exchanging sample related information SRI with the IVD laboratory control software module 3, and the proposed simulation method comprises
- exchanging sample related information SRI between the IVD laboratory control software module 3 and the data management system 6.

Connecting the IVD laboratory control software module 3 to a data management system 6 can allow testing the IVD laboratory control software module 3 and/or a certain configuration thereof in a realistic manner. An example of a real world data management unit 6 being connected to an IVD laboratory control software module 3 that is connected to a virtual IVD laboratory instrumentation 5 is depicted in Figs. 4, 6, and 8.

According to some embodiments, the method comprises
- simulating a virtual data management system 56 configured for exchanging sample related information SRI with the IVD laboratory control software module 3, and
- exchanging sample related information SRI between the IVD laboratory control software module 3 and the virtual data management system 56.

The schema of **Figs. 5****,** **7****,** and **9** each shows an example wherein the data management unit 6 is simulated as a virtual data management system 56 as part of the simulation software module 4. Using a virtual data management system 56 can allow testing data management system related functionalities of the IVD laboratory control software module 3 without connecting it to a real-world data management system 6. The virtual data management system 56 can be designed for simulating the operational behavior, in particular the communicational behavior, of the a data management unit 6. The virtual data management system 56 can e.g. be created using recordings of a real-world data management system's communicational behavior, e.g. by using an artificial intelligence unit that was trained using said recordings.

According to some specific embodiments, the method comprises
- receiving - by the IVD laboratory control software module 3 - a first data from the virtual data management system 56 or from the virtual IVD laboratory instrumentation 5, the first data comprising an indication that a sample has arrived at the IVD laboratory instrumentation 2, thereby receiving a virtual sample;
- receiving - by the IVD laboratory control software module 3 - a second data from the virtual data management system 56, the second data comprising a test order for the sample, thereby receiving a test order for the virtual sample;
- simulating sample processing of the virtual sample according to the test order using the virtual IVD laboratory instrumentation 5.

The time at which each of the first data and the second data are sent from the virtual data management system 56 resp. virtual IVD laboratory instrumentation 5 can be chosen or randomized, which can allow to simulating the IVD laboratory system's behavior when an sample arrives earlier at the IVD laboratory instrumentation 2 than the respective test order at the IVD laboratory control software module 3 - or vice versa. The first data can be received by the IVD laboratory control software module 3 from the virtual data management system 56 via the virtual IVD laboratory instrumentation 5, which can allow using the virtual data management system 56 as the source for both the indication that a sample has arrived as well as the test order.

According to some embodiments, an input file is used to provide to the simulation with information as to which samples are to be simulated as to be received and the respective test orders. According to an example, the input file comprises for each sample at least one of
- a receiving time of a sample,
- a test order for a sample,
- a receiving time of a test order,
- a volume of a sample, and
- test results for a test order.

The simulation software module 4 may be designed to receive the input file using the virtual data management system 56, e.g. wherein the virtual data management system 56 is the only software entity of the simulation software module 4 that access the information of the input file directly. The virtual data management system 56 may be designed to provide data created using the input file - optionally at least some of the information via the virtual IVD laboratory instrumentation 5 - to the IVD laboratory control software module 3. According to an example, the virtual data management system 56 sends a test order and a receiving time of the test order to the IVD laboratory control software module 3; the virtual data management system 56 causes the virtual IVD laboratory instrumentation 5 (e.g. a virtual pre-analytical IVD laboratory instrument 50pre thereof) to send an indication that a respective sample has arrived in the IVD laboratory instrumentation 2 to the IVD laboratory control software module 3 at the receiving time of the respective sample; the virtual data management system 56 provides to the virtual IVD laboratory instrumentation 5 a volume of the sample and/or test results for the test order for the sample to the virtual IVD laboratory instrumentation 5 and the virtual IVD laboratory instrumentation 5 sends - possibly in reply to respective instructions by the IVD laboratory control software module 3 - sample processing data SPD to the IVD laboratory control software module 3, wherein the sample processing data SPD is created/chosen using the volume of the sample and/or the test results for test order of the sample.

According to some embodiments, the virtual IVD laboratory instrumentation 5 comprises a virtual IVD laboratory instrument 50 that is designed for simulating an IVD laboratory instrument 20, e.g. by simulating its operational behavior. Simulating the operational behavior can in particular comprise simulating receiving samples, simulating processing samples, and/or receiving/creating/sending sample processing data SPD.

Simulating the operational behavior can in particular comprise simulating the communicational behavior, e.g. the interaction of the IVD laboratory instrument 20 at its communication interfaces. The virtual IVD laboratory instrument 50 can be designed for simulating the communicational behavior of a IVD laboratory instrument 20 in that it is designed for replying to sample processing data SPD it receives from the IVD laboratory control software module 3 in a similar way, e.g. essentially in the same way, as the respective IVD laboratory instrument 20 would. According to some embodiments, the virtual IVD laboratory instrument 50 is designed for sending the same kind of sample processing data SPD in the same data format as a respective IVD laboratory instrument 20; the virtual IVD laboratory instrument 50 can further be designed for creating sample processing data SPD, e.g. by using an algorithm to create the content of the data and/or by copying sample processing data SPD being stored in the virtual IVD laboratory instrument 50. The time difference between receiving a certain sample processing data SPD from the IVD laboratory control software module 3 and replying to this sample processing data SPD by sending responsive sample processing data SPD can be similar to a time difference that would occur for an IVD laboratory instrument 20 in the same situation, which can e.g. be realized by recording the timings of the communications of a real IVD laboratory instrument 20 and then using this recordings to design the response timings of the virtual IVD laboratory instrument 50.

The type of response of the virtual IVD laboratory instrument 50 to a same type of sample processing data SPD can be vary from response to response, e.g. a reply to an instructions to conduct a certain test can in some of the cases be a respective test result and in some of the cases be indications of various types of errors (that e.g. have occurred during conducting this test in a respective reference IVD laboratory instrument 20). The timings of the error indications can e.g. depend on the type of error, e.g. a fast response for an error that typically occurs early in the testing workflow and a slow response for an error that typically occurs late in the testing workflow. In general, the timings of sending sample processing data SPD by the virtual IVD laboratory instrument 50 to the IVD laboratory control software module 3 can be subject to variance using statistical distributions, which can e.g. allow for simulating realistic timings.

Each virtual IVD laboratory instrument 50 can be realized as an independent software entity, wherein the operational behavior of the each virtual IVD laboratory instrument 50 is calculated independently of the other virtual IVD laboratory instruments 50, e.g. wherein, except for possible sample processing data SPD exchanged via the IVD laboratory control software module 3, no parameter of the operational behavior of a first virtual IVD laboratory instrument 50 is used for calculating the operational behavior of a second virtual IVD laboratory instrument 50. If the simulation comprises a visualization (e.g. a graphic display of a schema of the IVD laboratory instrumentation 2 to be simulated, possibly comprising a graphic display indicating the samples at locations where they are currently processed according the simulation), each virtual IVD laboratory instrument 50 can be displayed in the visualization.

A method for designing a virtual IVD laboratory instrument 50 for simulating an IVD laboratory instrument 20 for use in a simulation method (e.g. as proposed herein) is proposed; the method of designing comprising
- recording and/or defining (e.g. creating) data files, e.g. sample processing data SPD files, being received and/or being sent by the IVD laboratory instrument 20, and/or
- recording and/or defining (e.g. creating) time gaps between the receiving and/or the sending of the data files, e.g. sample processing data SPD files, by the IVD laboratory instrument 20; and
- using the recorded resp. defined data files and/or the recorded resp. defined time gaps for creating the content and/or for defining the sending timing of data files, e.g. sample processing data SPD files, by the virtual IVD laboratory instrument 50.

This method of designing can in particular comprise recording all communication input and output and respective timings of a real-world IVD laboratory instrument 20, which can be used for designing a respective virtual IVD laboratory instrument 50. Recording the data files can allow creating a virtual IVD laboratory instrument 50 simulating a typical behavior of an IVD laboratory instrument 20; defining the data files can allow simulating special case of behavior, e.g. for testing the behavior of the IVD laboratory control software module 3 in special cases. Creating the virtual IVD laboratory instrument 50, in particular its communicational behavior, can comprise using an artificial intelligence module that has been trained using the recorded data files resp. time gaps. Creating the virtual IVD laboratory instrument 50 can take into consideration the type of test to be simulated by the virtual IVD laboratory instrument 50, the type of IVD laboratory instrument 20 to be to be simulated by the virtual IVD laboratory instrument 50, and/or specific workflow parameters (e.g. as typically or ideally used by the IVD laboratory instrument 20 to be to be simulated by the virtual IVD laboratory instrument 50)

According to some embodiments, virtual IVD laboratory instrumentation 5 can comprise multiple virtual IVD laboratory instruments 50 of various types for simulating the various steps of sample processing e.g. based on sample processing data SPD provided by the IVD laboratory control software module 3.

The virtual IVD laboratory instrumentation 5 can comprise one or more virtual IVD laboratory instruments 50. According to some embodiments, the virtual IVD laboratory instrumentation 5 comprises exactly one virtual IVD laboratory instrument 50 for each IVD laboratory instrument 20 of the IVD laboratory instrumentation 2 to be simulated, e.g. of an existing IVD laboratory instrumentation 2 or of a planned IVD laboratory instrumentation 2.

According to some specific embodiments, each virtual IVD laboratory instrument 50 simulates the operational behavior, in particular the communicational behavior, of the respective IVD laboratory instrument 20; according to an example, a virtual IVD laboratory instrument 50 simulating a specific pre-analytical IVD laboratory instrument 20pre can be designed for simulating the operational behavior of this specific pre-analytical IVD laboratory instrument 20pre.

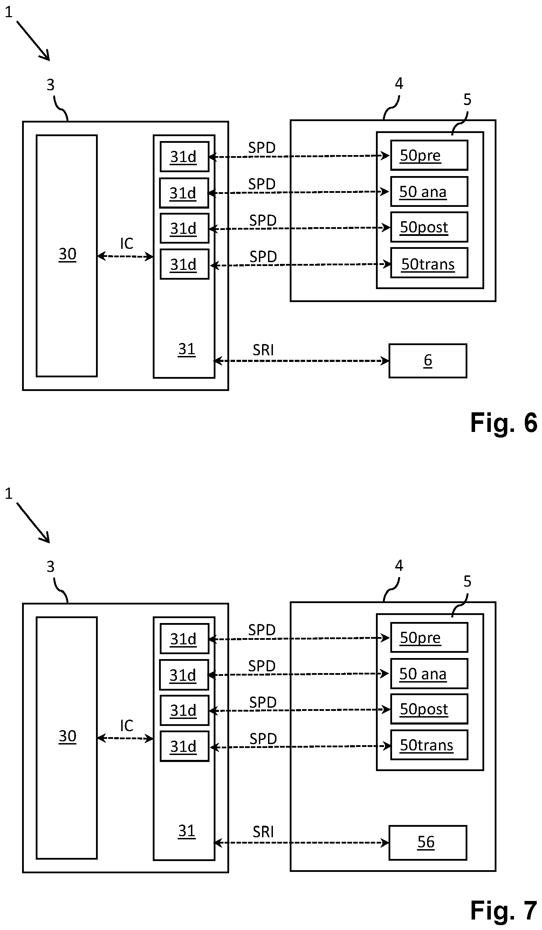

As displayed in the example of **Figs. 6 to 9** **and 11,** the virtual IVD laboratory instrumentation 5 can comprise
- a virtual pre-analytical IVD laboratory instrument 50pre designed for simulating (e.g. the operational behavior of) a pre-analytical IVD laboratory instrument 20pre;
- a virtual analytical IVD laboratory instrument 50ana designed for simulating a (e.g. the operational behavior of) analytical IVD laboratory instrument 20ana;
- a virtual post-analytical IVD laboratory instrument 50post designed for simulating (e.g. the operational behavior of) a post-analytical IVD laboratory instrument 20post; and/or
- a virtual transport IVD laboratory instrument 50trans designed for simulating (e.g. the operational behavior of) a transport IVD laboratory instrument 20trans.

According to some embodiments, the virtual IVD laboratory instrumentation 5 is designed for simulating the operational, e.g. communicational, behavior of an IVD laboratory instrumentation 2. According to some embodiments, the virtual IVD laboratory instrumentation 5 is designed for the simulating the communicational behavior of the IVD laboratory instrumentation 2 as such, wherein the simulation can e.g. be based on an artificial intelligence module trained using the overall communicational input and output of the IVD laboratory instrumentation 2 from/to the IVD laboratory control software module 3. According to some embodiments, the virtual IVD laboratory instrumentation 5 is designed for the simulating the communicational behavior of the each IVD laboratory instrument 20 of the IVD laboratory instrumentation 2 by using respective virtual IVD laboratory instruments 50, wherein the creating of the each virtual IVD laboratory instrument 50 can e.g. be based on an artificial intelligence module trained using the communicational input and output of the respective IVD laboratory instrument 20 from/to the IVD laboratory control software module 3, wherein the communicational input and output of the virtual IVD laboratory instrumentation 5 from/to the IVD laboratory control software module 3 can e.g. be the collection of all communicational input and output of each virtual IVD laboratory instrument 50 from/to the IVD laboratory control software module 3.

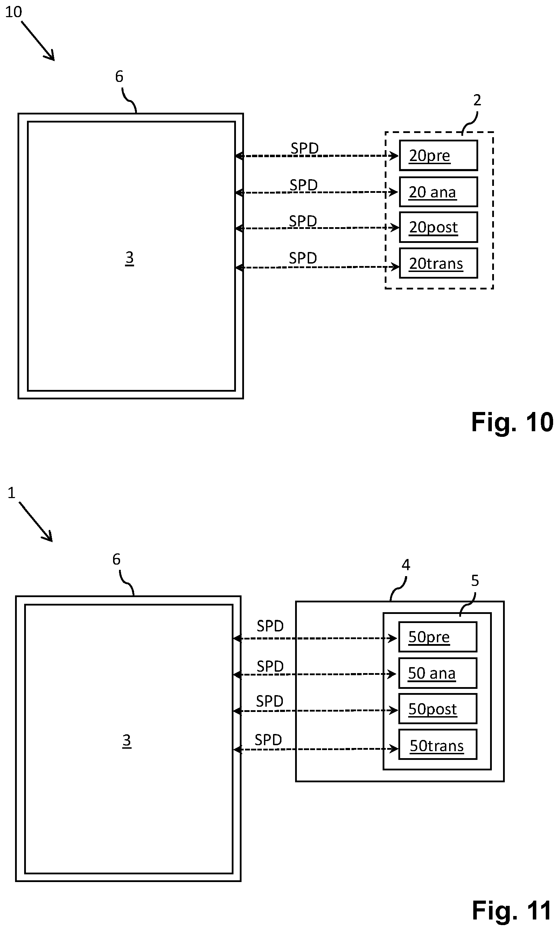

According to some embodiments, the IVD laboratory control software module 3 can be comprised in a data management unit 6, e.g. where an data management unit 6 is designed for controlling the sample processing within the laboratory. Examples of an IVD laboratory control software module 3 comprised in a data management unit 6 are illustrated in **FIG. 10** (real-world) and **Fig. 11** (simulation).

A system 1 for simulating an IVD laboratory system 10 for processing biological samples is proposed, wherein the IVD laboratory system 10 to be simulated comprises
- an IVD laboratory instrumentation 2 comprising one or more IVD laboratory instruments 20 designed for processing biological samples and
- an IVD laboratory control software module 3 designed for controlling sample processing within the IVD laboratory instrumentation 2 and exchanging sample processing data SPD between the IVD laboratory control software module 3 and the IVD laboratory instrumentation 2,
wherein the IVD laboratory system 10 to be simulated is designed for
- sample processing within the IVD laboratory instrumentation 2 using sample processing data SPD provided by the IVD laboratory control software module 3.

The proposed system 1 comprises
- the IVD laboratory control software module 3, and
- a simulation software module 4 comprising a virtual IVD laboratory instrumentation 5 for simulating sample processing within the IVD laboratory instrumentation 2;
wherein the system 1 is configured for
- operating the IVD laboratory control software module 3 as if it was operating within the IVD laboratory system 10, but wherein sample processing data SPD intended for being exchanged between the IVD laboratory control software module 3 and the IVD laboratory instrumentation 2 is instead exchanged between the IVD laboratory control software module 3 and the virtual IVD laboratory instrumentation 5;
wherein the virtual IVD laboratory instrumentation 5 is configured for
- simulating sample processing within the IVD laboratory instrumentation 2 using sample processing data SPD provided by the IVD laboratory control software module 3.

The system can in particular be realized as a software system, whose components can e.g. be realized by being executed by one or more processing units.

The simulation software module 4 can comprise a virtual IVD laboratory instrumentation 5 in that the simulation software module 4 comprises one or more virtual IVD laboratory instruments 50.

The simulation software module 4 can e.g. be created using a simulation framework for simulating discrete events, such as e.g. *SIMIO Simulation Software* of *Simio LLC* as available on 01^{th} of October 2021. Within such simulation framework, types of virtual IVD laboratory instruments 50 can be created that each represent a type of an IVD laboratory instrument 20, wherein each type of virtual IVD laboratory instruments 50 can e.g. be implemented using modules of the simulation framework, wherein the modules' properties are configured for representing (e.g. imitating) properties of the respective type of IVD laboratory instrument 20; the properties can e.g. concern the types of input and/or output of sample processing data SPD and relative timings of respective sample processing data SPD (e.g. to imitate the time needed for certain processes within this type of IVD laboratory instrument 20). A graphical user interface can be used for placing (e.g. by drag-and-drop) visual representations of the virtual IVD laboratory instruments 50 within a virtual IVD laboratory to create the virtual IVD instrumentation 5. Virtual transport IVD laboratory instruments 50trans can be used for representing connections for IVD container transporting; the virtual length and/or geometry of which can e.g. be used for calculating relative timings of respective sample processing data SPD representing transporting times between IVD laboratory instruments 20. The interfaces for and the details of the communication between the simulation software module 4 and the IVD laboratory control software module 3 can e.g. implemented within the simulation framework using program code (e.g. written in C# or a similar coding language); the simulation software module 4 can in particular be designed to process incoming sample processing data SPD and/or create outgoing sample processing data SPD using said program code within the simulation framework. Specifics, in particular of the IVD laboratory instrumentation's communicational behavior, can be taught to the simulation software module 4 by e.g. configuring functions provided by the simulation framework or by program code (e.g. at interfaces provided by the simulation framework); the steps to be performed for a specific test order for a sample may e.g. be implemented using logical flow charts within the simulation framework and the simulation framework - during execution - may e.g. call respective program code to cause the communicational behavior adequate for the respective step.

According to some embodiments, the IVD laboratory control software module 3 comprises a first communication module 31 comprising at least one driver 31d designed for transforming a data format of a data file (e.g. a data file comprising sample processing data SPD); some examples of such embodiments can be seen in Figs. 6 to 9. The drivers 31d can be designed for transforming data formats of sample processing data SPD to be exchanged between the IVD laboratory control software module 3 on the one side and the virtual IVD laboratory instrumentation 5 resp. the individual virtual IVD laboratory instruments 50 thereof on the other side. The drivers 31d of the communication module 31 used in the proposed simulation can e.g. be the same drivers used by the communication module 31 for interacting with a real-world IVD laboratory instrumentation 2 that is mimicked using the virtual IVD laboratory instrumentation 5; this can e.g. allow testing of drivers to be used with such IVD laboratory instrumentation 2. In case a virtual IVD laboratory instrument 50 simulates a specific IVD laboratory instrument 20, the driver 31d used for communication between the IVD laboratory control software module 3 and this virtual IVD laboratory instrument 50 can e.g. be the same driver as used for communication between the IVD laboratory control software module 3 and this specific IVD laboratory instrument 20.

According to some embodiments, the first communication module 31 for each virtual IVD laboratory instrument 50 comprises at least one driver for transforming data formats compatible with the virtual IVD laboratory instrument 50 to/from data formats compatible with the IVD laboratory control software module 3; this can e.g. allow the IVD laboratory control software module 3 to communicate with each virtual IVD laboratory instrument 50 of the virtual IVD laboratory instrumentation 5.

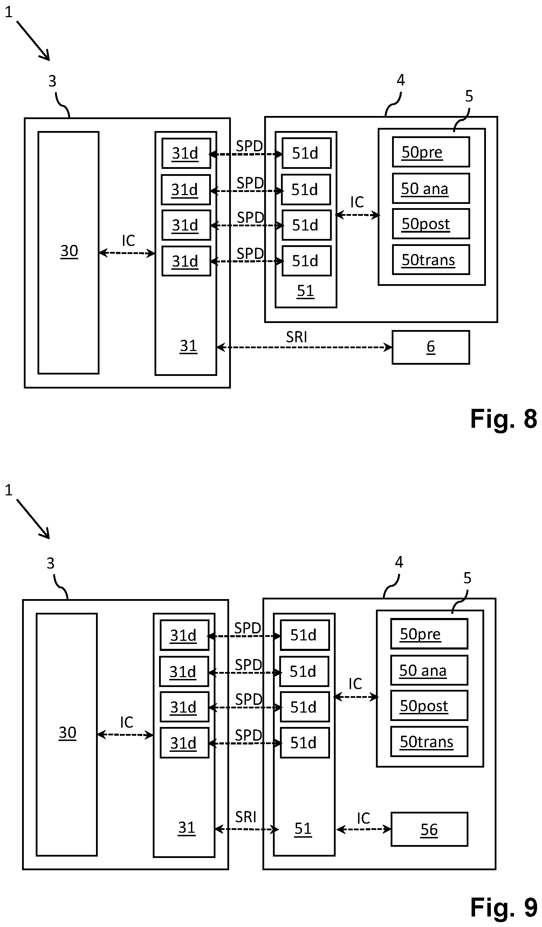

According to some embodiments, the simulation software module 4 comprises a second communication module 51 comprising at least one driver 51d for transforming a data format of a data file (e.g. a data file comprising sample processing data SPD); examples of such embodiments are illustrated in Figs. 8 and 9. According to an example, the IVD laboratory control software module 3 provides to the virtual IVD laboratory instrumentation 5 sample processing data SPD in one or more data formats that at least some of the virtual IVD laboratory instruments 50 cannot process; using suitable drivers 51d can allow the IVD laboratory control software module 3 to communicate with these virtual IVD laboratory instruments 50 in the intended manner. This can e.g. allow using virtual IVD laboratory instruments 50 using uniform data formats, e.g. the same data format the IVD laboratory control software module 3 uses internally, which can allow reducing the complexity of the various virtual IVD laboratory instruments 50.

According to some embodiments, the first communication module 31 and the second communication module 51 each comprise at least one joint driver 31d, 51d, e.g. wherein the joint driver 31d, 51d is bi-directional, i.e. it can transform from a first data format to a second data format and vice versa. According to some specific embodiments, the operation of the first communication module 31 is mirrored by the second communication module 51, e.g. the data format in which the first communication module 31 receives data file from the control components 30 is identical to the data format in which the second communication module 51 provides a data file to the virtual IVD laboratory instruments 50. In particular, each driver of the first communication module 31 and the second communication module 51 can be a joint driver 31d, 51d used by both communication modules 31, 51. This can allow the virtual IVD laboratory instruments 50 to use the same data format as the IVD laboratory control software module 3 uses for its internal communication IC, so that - for a given IVD laboratory control software module 3 - only virtual IVD laboratory instruments 50 using that data format must be created, while it is still possible to simulate the behavior of the IVD laboratory control software module 3 with IVD laboratory instruments 20 that use other data formats.

According to some embodiments, the sample processing data SPD is indicative of (e.g. comprises) test orders, e.g. an indication as to which analytical tests to be performed on an individual samples. The IVD laboratory control software module 3 can be designed for determining at least some of the sample processing steps for conducting test orders, e.g. by using a databank whose entries link test orders to respective sample processing steps. The IVD laboratory control software module 3, in particular a workflow engine 30w of the IVD laboratory control software module 3, can comprise an optimization module designed for optimizing in which order and by which IVD laboratory instruments 20 the sample processing steps of multiple test orders for multiples samples shall be performed. Optionally, the test order can further comprise an indication of an urgency of the sample. The IVD laboratory control software module 3 can be designed to prioritize the sample processing steps to be performed on a sample according to the urgency of an sample, e.g. by taking the urgency of a sample into account for an optimization of the sample processing steps of multiple test orders for multiples samples.

According to some embodiments, the sample processing data SPD is indicative of (e.g. comprises) processing queries, e.g. an indication to the IVD laboratory control software module 3 that a specific IVD laboratory instrument 20 has received a specific sample and that the specific IVD laboratory instrument 20 requests an instruction on how to process this specific sample.

According to some embodiments, the sample processing data SPD is indicative of (e.g. comprises) processing orders, e.g. an indication by the IVD laboratory control software module 3 to the IVD laboratory instrumentation 2 how a specific sample should be processed (e.g. which analytical test(s) are to be performed for this sample). A processing order can be sent in reaction to a processing query.

According to some embodiments, the sample processing data SPD is indicative of (e.g. comprises) test results, e.g. test results of analytical tests performed on one or more analytical IVD laboratory instruments 20ana; the test result e.g. being a quantitative result (e.g. a glucose concentration in the sample) or a qualitative result (e.g. an indication that a certain virus type is present in the sample).

According to some embodiments, the sample processing data SPD is indicative of (e.g. comprises) transport queries, e.g. a query from an IVD laboratory instrument 20 to the IVD laboratory control software module 3 as to where a sample that is currently located at the IVD laboratory instrument 20 is to be transported next.

According to some embodiments, the sample processing data SPD is indicative of (e.g. comprises) transport orders, e.g. an indication from the IVD laboratory control software module 3 to an IVD laboratory instrument 20 as to where a sample that is currently located at the IVD laboratory instrument 20 is to be transported next. A transport order can be sent in reaction to a transport query.

According to some embodiments, the sample processing data SPD is indicative of (e.g. comprises) instrument status, e.g. that a specific IVD laboratory instrument 20 is powered and operational or that a specific IVD laboratory instrument 20 is masked.

According to some embodiments, the sample processing data SPD is indicative of (e.g. comprises) workflow instructions, e.g. an instruction concerning a specific workflow within the IVD laboratory instrumentation 2.

According to some embodiments, the sample processing data SPD is indicative of (e.g. comprises) positioning information, e.g. an indication concerning the current position of a sample within the IVD laboratory instrumentation 2 such as an indication at which IVD laboratory instrument 20 or at which submodule/section of an IVD laboratory instrument 20 the sample is currently located.

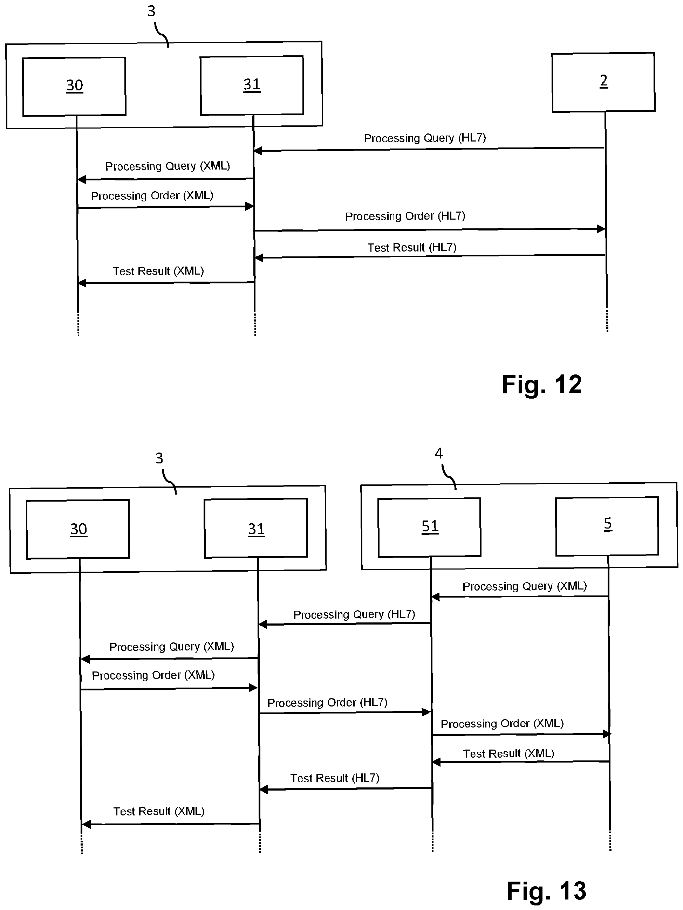

**Fig. 12** shows a scheme of a possible exchange of sample processing data SPD in an IVD laboratory system 10:
STEP A: The IVD laboratory instrumentation 2 creates a processing query for a specific sample and sends the processing query to the IVD laboratory control software module 3.
STEP B: The processing query is received by the communication module 31 and converted from a first data format, in this example "HL7", in which it was created to a second data format, in this example a "XML" file using a specific data structure, that is processible by the control components 30. The converted processing query is then sent to and processed by the control components 30. In reply to the processing query, the control components 30 creates a processing order in XML and sends this processing order to the communication module 31, where the processing order is converted to HL7.
STEP C: The processing order in HL7 is then sent to the IVD laboratory instrumentation 2.
STEP D: In the depicted example, the processing order indicates a specific analytical test to be performed on this specific sample, and after this specific analytical test has been performed on this specific sample, a test result in HL7 is created and sent from the IVD laboratory instrumentation 2 to the IVD laboratory control software module 3.
STEP E: The communication module 31 receives and converts the test result to XML and sends this converted test result to the control components 30. The control components 30 may then e.g. validate the test result and send it to a data management unit 6 (not depicted).

**Fig. 13** shows a scheme of a possible exchange of sample processing data SPD according to one of the proposed methods for simulating; the depicted example resembling the example of Fig. 12:
STEP A: Within the simulation software module 4, the virtual IVD laboratory instrumentation 5 creates a processing query for a specific virtual sample. In the depicted case, the processing query is initially created the XML. Although in the depicted example the control components 30 would be able to process the XML-file, the IVD laboratory control software module 3 in real-life is designed for receiving sample processing data SPD in HL7 and in order to simulate the data exchange with the IVD laboratory control software module 3 realistically, the processing order is sent to a virtual communication module 51 of the simulation software module 4 where the processing query is converted to HL7. The converted processing query is then sent to the IVD laboratory control software module 3
STEP B: As in the example of Fig. 12, the processing query is received by the communication module 31 and converted from HL7 to XML; the converted processing query in HL7 is then sent to and processed by the control components 30; in reply to the processing query, the control components 30 creates a processing order in XML and sends this processing order to the communication module 31, where the processing order is converted to HL7.
STEP C: The processing order in HL7 is then sent to the simulation software module 4 and received by the virtual communication module 51, where is it is converted to XML. The virtual communication module 51 might simply mirror the operation of the communication module 31, such that the processing order is now identical (or at least very similar) to the processing order originally created by the control components 30. The processing order in XML is then sent to the virtual IVD laboratory instrumentation 5.
STEP D: In analogy to the example of Fig. 12, the processing order in this example as well indicates a specific analytical test to be performed on this specific virtual sample. In reply to the processing order, the virtual IVD laboratory instrumentation 5 creates (e.g. using a databank of test results obtained by a respective real-world IVD laboratory instrumentation 2 for a processing order of the same type) a test result in XML and sends it to the virtual communication module 51, where the text result is converted to HL7 and sent to the IVD laboratory control software module 3. STEP E: As in the example of Fig. 12, the communication module 31 receives and converts the test result to XML and sends this converted test result to the control components 30. The control components 30 may then e.g. validate the test result and send it to a data management unit 6 or a virtual data management system 56 (not depicted).

As can be seen from comparing the respective STEPs B and E described above for the examples of Figs. 12 and 13, the IVD laboratory control software module 3 in both examples operates in exactly the same manner. In this sense, the IVD laboratory control software module 3 is agnostic of whether it interacts with a real-world IVD laboratory instrumentation 2 or a virtual IVD laboratory instrumentation 5.

According to some embodiments, the method comprises simulating samples processed in the IVD laboratory system 10 as virtual samples 52. Simulating the sample processing on the level of individual virtual samples can allow for a high degree of realism of the simulation.

According to some specific embodiments, the method comprises displaying a visualization of the virtual IVD laboratory instrumentation 5 together with a visualization of virtual samples 52, e.g. as resp. as part of virtual IVD containers and/or as resp. as part of virtual IVD container holders.

According to some specific embodiments, the virtual IVD laboratory instrumentation 5 is displayed as a schematic representation corresponding to the IVD laboratory instrumentation 2 to be simulated. According to some specific embodiments, the virtual samples 52, e.g. as resp. as part of virtual IVD containers and/or as resp. as part of virtual IVD container holders, each are displayed at a position in the schematic representation corresponding to a position of a corresponding sample being simulated at the time.

According to some embodiments, a schematic representation of a visualization of details of the design of the virtual IVD laboratory instrumentation 5 is graphically displayed, e.g. as part of a graphical user interface 9.

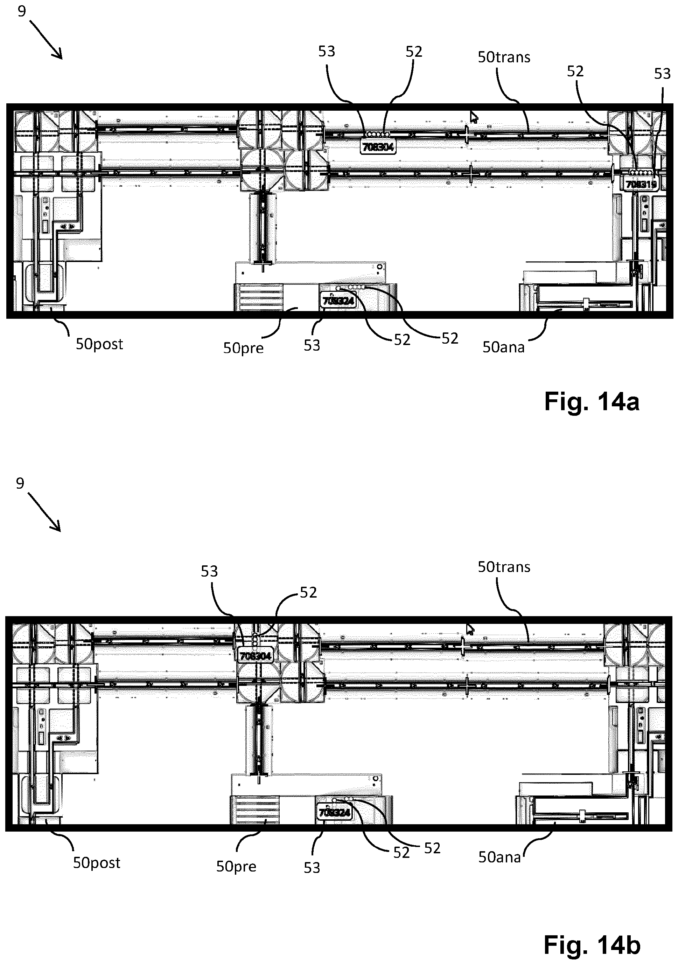

**Fig. 14a** shows a possible example of a graphical display, e.g. a part of a graphical user interface 9, visualizing the virtual IVD laboratory instrumentation 5 and the virtual samples 52 therein. In the depicted example, the virtual IVD laboratory instruments 50 are represented by an image resembling respective IVD laboratory instruments 20 from above and each virtual sample 52 is visualized by a circle to represent the shape of a sample tube from above. Of course, other perspectives are conceivable as well. The visualization shows virtual sample container holders 53, each capable of holding up to five sample tubes; the virtual sample container holders 53 are indexed by number, which may allow a spectator to differentiate between and track the individual virtual sample container holders 53. In Fig. 14a, three virtual sample container holders 53 are displayed:
- virtual sample container holder No. 708304 holds five samples 52 and is being transported via virtual transport IVD laboratory instrument 50trans;
- virtual sample container holder No. 708319 holds five samples 52 and is being located at an entrance of virtual analytical IVD laboratory instrument 50ana (only a part which being visible in the cutout depicted in Fig. 14a); and
- virtual sample container holder No. 708324 holds one sample 52 and is being located within virtual pre-analytic IVD laboratory instrument 50pre, wherein four samples 52 are being displayed next to it.

The visualization can be designed to fluidly adapt to the situation in the virtual IVD laboratory instrumentation 5 as it is currently simulated. **Fig. 14b** shows a possible example of the graphical display of Fig. 14a at a second time, wherein
- virtual sample container holder No. 708304 has been moved to the left and rotated by 90°;
- virtual sample container holders No. 708319 is no longer visible in the depicted cutout (e.g. because it has been moved to a part of the virtual analytical IVD laboratory instrument 50ana outside the depicted cutout); and
- virtual sample container holder No. 708324 is still being located within virtual pre-analytic IVD laboratory instrument 50pre, but now holds three samples 52 and two samples 52 are being displayed next to it (e.g. representing that in between two additional samples have been loaded into virtual sample container holder No. 708324).

Such a visualization can allow a spectator to monitor the simulated sample processing within the virtual IVD laboratory instrumentation 5, which in turn can e.g. allow to analyze the effectiveness and/or the weaknesses of the design of an IVD laboratory instrumentation 2 being simulated by the virtual IVD laboratory instrumentation 5 and/or allow for training personnel for working with the IVD laboratory system 10. The visualization can further comprise a visualization of personnel operation within the virtual IVD laboratory instrumentation 5, e.g. representing a staff member that walks to a first IVD laboratory instrument 20, withdraws a sample form the IVD laboratory instrumentation 2, and later reinserts the sample to the IVD laboratory instrumentation 2, possibly at a second IVD laboratory instrument 20; this can e.g. allow for testing personnel operation within the IVD laboratory system 10.

According to some embodiments, the method comprises receiving a virtual sample e.g. by simulating receiving a sample; this can e.g. be realized in that the virtual IVD laboratory instrumentation 5, a simulation of a respective IVD laboratory instrumentation 2, creates data being indicative of a virtual sample (e.g. based on an inputted file) and sends sample processing data SPD indicative of having received a sample to the IVD laboratory control software module 3, thereby announcing to the IVD laboratory control software module 3 that the sample has been received in the IVD laboratory instrumentation 2 that the IVD laboratory control software module 3 believes it is connected to. According to some specific embodiments, the method can further comprise receiving a test order for said virtual sample; this can e.g. be realized by sending the test order to the IVD laboratory control software module 3, e.g. by a data management system 6 being in communication with the IVD laboratory control software module 3 or by a virtual data management system 56 (which can e.g. be simulated as part of the virtual IVD laboratory instrumentation 5 or simulated independently of the virtual IVD laboratory instrumentation 5) being in communication with the IVD laboratory control software module 3. According to an example, the IVD laboratory control software module 3 being in communication with the virtual IVD laboratory instrumentation 5 receives a test order and an indication that a respective sample has been received by an IVD laboratory instrumentation 2 (that the virtual IVD laboratory instrumentation 5 simulates) and sends sample processing data SPD based on the test order to the virtual IVD laboratory instrumentation 5 as if the virtual IVD laboratory instrumentation 5 was a (real-world) IVD laboratory instrumentation 2, wherein the sample processing data SPD is designed so that this sample processing data SPD would cause the IVD laboratory instrumentation 2 to process the sample in accordance with the test order.

According to some embodiments, the method comprises simulating receiving of the virtual sample by the virtual IVD laboratory instrumentation 5 resp. a virtual IVD laboratory instrument 50 thereof.

According to some embodiments, the method comprises sending a processing query for a virtual sample from the virtual IVD laboratory instrumentation 5 resp. a virtual IVD laboratory instrument 50 thereof to the IVD laboratory control software module 3.

According to some embodiments, the method comprises sending, e.g. after having received a processing query, a processing order for a virtual sample generated using a test order for this virtual sample from the IVD laboratory control software module 3 to the virtual IVD laboratory instrumentation 5 resp. a virtual IVD laboratory instrument 50 thereof.

According to some embodiments, the method comprises simulating sample processing in the form of sample testing, e.g. according to a test order the virtual IVD laboratory instrumentation 5 resp. a virtual IVD laboratory instrument 50 thereof, e.g. by sending a (e.g. created and/or received) test result from the virtual IVD laboratory instrumentation 5 resp. a virtual IVD laboratory instrument 50 thereof to the IVD laboratory control software module 3. The test result can be sent with a time gap, e.g. relative to the start of the simulation of the sample testing, wherein the time gap is indicative of a time needed for this sample testing in the IVD laboratory instrumentation 2 resp. an IVD laboratory instrument 20 thereof.

According to some embodiments, the method comprises evaluating, e.g. validating, a received test result by the IVD laboratory control software module 3.

According to some embodiments, the method comprises sending a transport query for a virtual sample from the virtual IVD laboratory instrumentation 5 resp. a virtual IVD laboratory instrument 50 thereof to the IVD laboratory control software module 3.

According to some embodiments, the method comprises sending, e.g. after having received a transport query, a transport order for a virtual sample from the IVD laboratory control software module 3 to the virtual IVD laboratory instrumentation 5 resp. a virtual IVD laboratory instrument 50 thereof.

According to some embodiments, the method comprises simulating a transport of the virtual sample within the virtual IVD laboratory instrumentation 5, e.g. according to a transport order.

According to some embodiments, the method comprises sending positioning information from the virtual IVD laboratory instrumentation 5 resp. a virtual IVD laboratory instrument 50 thereof to the IVD laboratory control software module 3, the positioning information concerning a current simulated position of a virtual sample 52 within the virtual IVD laboratory instrumentation 5 resp. a virtual IVD laboratory instrument 50 thereof.

According to some embodiments, the method comprises exchanging quality control ("QC") data between the IVD laboratory control software module 3 and the virtual IVD laboratory instrumentation 5, e.g. wherein said QC data is intended for being exchanged between the IVD laboratory software 3 and the IVD laboratory instrumentation 2 in the IVD laboratory system 10. According to some specific embodiments, the method comprises sending QC data from the virtual IVD laboratory instrumentation 5 to the IVD laboratory control software module 3. According to an example, the QC data comprise data identical or similar to test results as well as information that classifies this data as QC results. The IVD laboratory control software module 3 can be configured to handle QC results differently than test results, e.g. by using information that classifies data identical or similar to test results as QC results. According to some embodiments, the simulation comprises creating QC data, e.g. according to pre-defined rules. The format of created QC data can be chosen to be identical to the format that is used for QC data in the IVD laboratory system 10 to be simulated.

According to some embodiments, the method comprises simulating sample processing by personnel within the IVD laboratory system 10, which can e.g. comprise
- simulating the manual workflow such as putting samples into and/or removing samples from the IVD laboratory instrumentation,
- simulating walking routes of personnel, and/or
- scheduling of working pauses of personnel.

According to some embodiments, the design of the virtual IVD laboratory instrumentation 5 is created using a design file. The design file can be a file that can be used for planning the layout of an IVD laboratory instrumentation 2; according to an example the design file comprises
- the definition of the IVD laboratory instruments 20 in the IVD laboratory instrumentation 2 and
- the details of the placement of the IVD laboratory instruments 20 and/or the connections between the IVD laboratory instruments 20 in the IVD laboratory instrumentation 2.

The design file can e.g. be or comprise a Visio file (.VSD). According to some specific embodiments, the details of the design of the virtual IVD laboratory instrumentation 5 are automatically created using the design file, e.g. by applying a set of rules to the information comprised in the design file.

According to some embodiments, the design of the virtual IVD laboratory instrumentation 5 is created using a list of IVD laboratory instruments 20 defined in the IVD laboratory control software module 3. According to some specific embodiments, the details of the design of the virtual IVD laboratory instrumentation 5 is automatically created using the list of IVD laboratory instruments 20 defined in the IVD laboratory control software module 3, e.g. by applying a set of rules to the list. According to an example, the design is created in that the IVD laboratory instruments 20 of the list are placed into the design and connected to one another according to certain rules.

According to some embodiments, the design of the virtual IVD laboratory instrumentation 5 are created using a graphical user interface 9. According to some specific embodiments, the graphical user interface 9 comprises a schematic representation of a visualization of details of the design of the virtual IVD laboratory instrumentation 5. According to an example, the graphical user interface 9 comprises a drag-and-drop functionality; the drag-and-drop functionality e.g. can allow to drag-and-drop images representing IVD laboratory instruments 20 or other parts of an IVD laboratory instrumentation 2 within the graphical user interface 9. Using an graphical user interface 9 can allow creating the virtual IVD laboratory instrumentation's design from scratch or modifying on an existing design, e.g. a design that was created using a design file and/or a list of IVD laboratory instruments 20 defined in the IVD laboratory control software module 3.

According to some embodiments,
- simulating sample processing within the IVD laboratory instrumentation 2 and
- operating the IVD laboratory control software module 3,
are both performed in real-time. Typically simulations are run much faster than real-time so that long-term behavior can be tested in relatively short time. However, operating the proposed simulation in real-time can allow for mimicking the operation of the IVD laboratory system 10 more realistically, thereby increasing the significance of a testing, e.g. a testing of the IVD laboratory control software module 3 and/or a certain configuration thereof.

According to some embodiments, the simulation is designed to run at various speeds, e.g. faster than real-time (e.g. at least up to ten times as fast) and/or slower than real-time (e.g. at least lower than half as fast).

According to some embodiments, the method comprises the following steps:
- simulating sample processing within the IVD laboratory instrumentation 2 in a simulation time;
- operating the IVD laboratory control software module 3 in real-time;
- comparing the time passed in the simulation time to the real-time;
- and:
   ∘ increasing the speed of the simulation time if the simulation time is behind the real-time (e.g. more than 1 second behind),
   ∘ decreasing the speed of the simulation time if the simulation time is ahead of the real-time (e.g. more than 1 second ahead), or
   ∘ maintaining the speed of the simulation time if the simulation time is at least quasi-identical to the real-time (e.g. if the two times differ by less than 1 second).

The comparison can be done regularly in certain measurement intervals (e.g. every second). Increasing the speed of the simulation time if the simulation time is behind the real-time can e.g. be done proportionally to the observed time gap (or proportionally to the observed time gap divided by the length of a measurement interval). Decreasing the speed of the simulation time if the simulation time is ahead the real-time can e.g. be done proportionally to the observer time gap (or proportionally to the observed time gap divided by the length of a measurement interval).

The proposed methods of simulating by operating the IVD laboratory control software module 3 in connection with a simulated IVD laboratory can allow to support
- designing an IVD laboratory instrumentation 2, an IVD laboratory control software module 3, and/or an IVD laboratory system 10;
- testing (in particular benchmarking) an IVD laboratory instrumentation 2, an IVD laboratory control software module 3, and/or an IVD laboratory system 10
- testing personnel operation within an IVD laboratory system 10;
- training personnel for working in an IVD laboratory system 10 (in particular for using the IVD laboratory control software module 3).

A method for designing an IVD laboratory control software module 3 using one of the proposed methods for simulating is proposed, the method for configuring comprising the following steps in the following order:
- STEP 0: providing a design of the IVD laboratory control software module 3;
- STEP 1: performing the method for simulating;
- STEP 2: changing one or more parameters of the design of the IVD laboratory control software module 3 (e.g. a configuration parameter);
- STEP 3: performing STEP 1; and
- STEP 4: optionally repeating STEP 2 and STEP 3.

STEP 4 is optional and can e.g. be omitted if performing the method according to STEP 3 is deemed successful, e.g. based on a benchmarking. The designing an IVD laboratory control software module 3 can e.g. comprise creating the IVD laboratory control software module 3, adding modules and/or features to the IVD laboratory control software module 3, and/or configuring the IVD laboratory control software module 3. In practice, configuring an IVD laboratory control software module 3 at an installed IVD laboratory instrumentation 2 can take a lot of time (up to multiple months) during which the IVD laboratory system 10 may (at least in part) be unusable. Being able to configure an IVD laboratory control software module 3 using a virtual IVD laboratory instrumentation 5 resembling the IVD laboratory instrumentation 2 can allow reducing the down-time of the IVD laboratory instrumentation 2 significantly and thus may allow to avoid unnecessary waste of testing capabilities and other resources.

A method for designing an IVD laboratory instrumentation 2 using one of the proposed methods for simulating is proposed, the method for designing comprising the following steps in the following order:
- STEP 0: providing a design of the IVD laboratory instrumentation 2;
- STEP 1: performing the method for simulating;
- STEP 2: changing one or more parameter of the design of the IVD laboratory instrumentation (2);
- STEP 3: performing STEP 1; and
- STEP 4: optionally repeating STEP 2 and STEP 3.

STEP 4 is optional and can e.g. be omitted if performing the method according to STEP 3 is deemed successful, e.g. based on a benchmarking. Designing an IVD laboratory instrumentation 2 can be a complex task; simulating an IVD laboratory instrumentation according to chosen design and testing its performance in cooperation with an IVD laboratory control software module 3 can allow determining the feasibility and/or the efficiency of the chosen design, therefore possibly allowing improving the design using the proposed method for simulating.

A method for designing an IVD laboratory system 10 using one of the proposed methods for simulating is proposed, the method for designing comprising the following steps in the following order:
- STEP 0: providing a design of the IVD laboratory control software module 3 and a design of the IVD laboratory instrumentation 2;
- STEP 1: performing the method for simulating;
- STEP 2: changing one or more parameter of the design of the IVD laboratory control software module 3 (e.g. a configuration parameter) and/or of the design of the IVD laboratory instrumentation 2 (e.g. a specific type of an IVD laboratory instruments 20 thereof);
- STEP 3: performing STEP 1; and
- STEP 4: optionally repeating STEP 2 and STEP 3.

STEP 4 is optional and can e.g. be omitted if performing the method according to STEP 3 is deemed successful, e.g. based on a benchmarking.

According to some embodiments, the method for designing an IVD laboratory instrumentation 2, an IVD laboratory control software module 3, and/or an IVD laboratory system 10 may comprise that STEP 1 comprises benchmarking the performance of the IVD laboratory control software module 3 and/or the IVD laboratory instrumentation 2. A possible parameter for the benchmarking is the so-called turn-around-time ("TAT"), i.e. the time the processing of a sample needs; possible benchmark parameters can e.g. be the average TAT of all sample and/or the average TAT of certain sample, e.g. high priority samples. Benchmarking can allow comparing designs, which in turn can allow improving/optimizing a design.

A method for testing an IVD laboratory instrumentation 2, an IVD laboratory control software module 3, and/or an IVD laboratory system 10 using one of the proposed methods for simulating is proposed. Such a method for testing can e.g. support creating documentations and/or satisfying regulatory requirements.

A method for testing personnel operation of an IVD laboratory system 10 using one of the proposed methods for simulating is proposed. This can allow to support personnel optimization, defining personnel roles, personnel planning, schedule planning, pause planning, etc.

A method for training personnel for working in an IVD laboratory system 10 using one of the proposed methods for simulating is proposed. According to some embodiments, the method comprises training personal for using the IVD laboratory control software module 3. This can allows training the personnel without the live IVD laboratory system 10, e.g. before the IVD laboratory system 10 is operational or to avoid problem in the operation of the IVD laboratory system 10 due to mistakes made during the training. In particular, this can allow training the personnel to deal with problematic situations without creating such a problematic situations for a live IVD laboratory system 10.

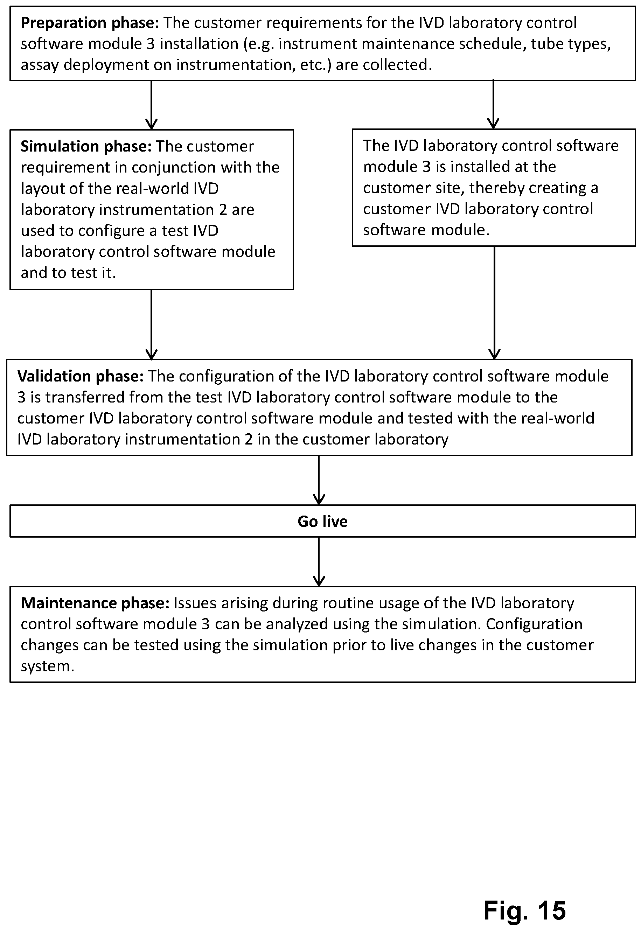

**Fig. 15** shows possible use cases of the proposed methods for a new installation of an IVD laboratory control software module 3, e.g. for an existing IVD laboratory instrumentation 2.
- In a preparation phase, the customer requirements for the IVD laboratory control software module 3 installation (e.g. instrument maintenance schedule, tube types, assay deployment on instrumentation, etc.) are collected.
- In a simulation phase, a simulation software module 4 comprising a virtual IVD laboratory instrumentation 5 resembling the customer's IVD laboratory instrumentation 2 is created and connected to a test IVD laboratory control software module 3, thereby creating a software system 1 that can be used according to the proposed methods. This can allow to configure the IVD laboratory control software module 3 in accordance to the customer requirements and to test the operation of the configured IVD laboratory control software module 3 with the customer's IVD laboratory instrumentation 2. This does not require a field service engineer to visit the customer site of the customer, the existence of the customer's intended IVD laboratory instrumentation 2, and/or the existence of an IVD laboratory control software module 3 at the customer's site. Possibly in parallel to the simulation phase, an IVD laboratory control software module 3 can be installed at the customer site, thereby creating a customer IVD laboratory control software module 3.
- In a validation phase, the tested configuration of the IVD laboratory control software module 3 is transferred from the test IVD laboratory control software module to the customer IVD laboratory control software module and tested with the real-world IVD laboratory instrumentation 2 in the customer's IVD laboratory system 10. After the configuration is validated, the IVD laboratory system 10 can go live. Using the proposed method of simulating the IVD laboratory system 10 can allow for a significant reducing the overall time needed at the customer's site for configuring and validating the IVD laboratory control software module 3. This can allow for a faster installation of the IVD laboratory control software module 3 and/or less down-time of the IVD laboratory system 10, which can e.g. allow for a significant reduction of commercial losses and/or a reduction of waste of IVD capacities.

- In a maintenance phase, the proposed simulation can be used for analyzing issues, which may save down-time and/or may allow for a better analysis. Intended changes in the configuration of the IVD laboratory control software module 3 can be tested using the simulation prior to live changes in the customer systems, which as well may save down-time. Overall, using the proposed simulation can allow for a more efficient and higher quality customer service.

The proposed system 1 may be implemented using one or more computer programs, e.g. an first computer program for the IVD laboratory control software module 3 and a second computer program for the simulation software module 4. Each of the computer programs may be executed by one or more processing units that can e.g. be comprised in a control unit, a server, a personal computer, and/or a cloud system. Each processing unit may comprise a processor, a volatile memory, a non-volatile memory, and a bus structure connecting these components.

The proposed methods may - at least partially - be implemented as computer-implemented methods e.g. to be performed by one or more processing unit executing respective software components.

Computer programs comprising instructions to cause one or more processing units to execute one or more of the proposed methods are proposed. Furthermore, computer-readable data carriers comprising said computer programs are proposed.

Systems 1, in particular software systems, that are designed for performing any of the proposed methods are proposed.

Methods embodied by any of the proposed systems 1 are proposed.

In particular, the following proposals are proposed:
Proposal 1: A method for simulating an in-vitro diagnostic IVD laboratory system 10 for processing biological samples, the IVD laboratory system 10 comprising
   - an IVD laboratory instrumentation 2 comprising one or more IVD laboratory instruments 20 for processing samples,
   - an IVD laboratory control software module 3 for controlling sample processing within the IVD laboratory instrumentation 2 and exchanging sample processing data SPD between the IVD laboratory control software module 3 and the IVD laboratory instrumentation 2,
   wherein the IVD laboratory system 10 is designed for
   - sample processing within the IVD laboratory instrumentation 2 using sample processing data SPD provided by the IVD laboratory control software module 3;
   the method comprising the steps of:
   - simulating sample processing within the IVD laboratory instrumentation 2 using a virtual IVD laboratory instrumentation 5 using sample processing data SPD provided by the IVD laboratory control software module 3,
   - operating the IVD laboratory control software module 3 as if it was operating within the IVD laboratory system 10 but wherein sample processing data SPD intended for being exchanged between the IVD laboratory control software module 3 and the IVD laboratory instrumentation 2 is instead exchanged between the IVD laboratory control software module 3 and the virtual IVD laboratory instrumentation 5.
Proposal 2: The method according to one of the preceding proposals, wherein the sample processing data SPD is indicative of test orders, processing queries, processing orders, test results, transport queries, transport orders, instrument status, workflow instructions, and/or positioning information.
Proposal 3: The method according to one of the preceding proposals, wherein the method comprises
   - receiving a virtual sample and a receiving a test order for the virtual sample;
   - simulating receiving of the virtual sample by the virtual IVD laboratory instrumentation 5 resp. a virtual IVD laboratory instrument 50 thereof;
   - sending a processing query for a virtual sample from the virtual IVD laboratory instrumentation 5 resp. a virtual IVD laboratory instrument 50 thereof to the IVD laboratory control software module 3;
   - sending, e.g. after having received a processing query, a processing order for a virtual sample generated using a test order for this virtual sample from the IVD laboratory control software module 3 to the virtual IVD laboratory instrumentation 5 resp. a virtual IVD laboratory instrument 50 thereof;
   - simulating sample processing in the form of sample testing, e.g. according to a test order the virtual IVD laboratory instrumentation 5 resp. a virtual IVD laboratory instrument 50 thereof, e.g. by sending a, e.g. created and/or received, test result from the virtual IVD laboratory instrumentation 5 resp. a virtual IVD laboratory instrument 50 thereof to the IVD laboratory control software module 3,
      ∘ e.g. wherein the test result is sent with a time gap from the start of the simulation of the sample testing, wherein the time gap is indicative of a time needed for this sample testing in an IVD laboratory instrumentation 2 resp. an IVD laboratory instrument 20 thereof;
   - evaluating a received test result by the IVD laboratory control software module 3;
   - sending a transport query for a virtual sample from the virtual IVD laboratory instrumentation 5 resp. a virtual IVD laboratory instrument 50 thereof to the IVD laboratory control software module 3;
   - sending, e.g. after having received a transport query, a transport order for a virtual sample from the IVD laboratory control software module 3 to the virtual IVD laboratory instrumentation 5 resp. a virtual IVD laboratory instrument 50 thereof;
   - simulating a transport of the virtual sample within the virtual IVD laboratory instrumentation 5, e.g. according to a transport order, and/or
   - sending positioning information from the virtual IVD laboratory instrumentation 5 resp. a virtual IVD laboratory instrument 50 thereof to the IVD laboratory control software module 3, the positioning information concerning a current simulated position of a virtual sample 52 within the virtual IVD laboratory instrumentation 5 resp. a virtual IVD laboratory instrument 50 thereof.
Proposal 4: The method according to one of the preceding proposals, wherein the method comprises exchanging quality control data between the IVD laboratory control software module 3 and the virtual IVD laboratory instrumentation 5.
Proposal 5: The method according to one of the preceding proposals, wherein the method comprises simulating sample processing by personnel within the IVD laboratory system 10.
Proposal 6: The method according to one of the preceding proposals, wherein the virtual IVD laboratory instrumentation 5 comprises exactly one virtual IVD laboratory instrument 50 for each IVD laboratory instrument 20 of the IVD laboratory system 10 to be simulated.
Proposal 7: The method according to of the preceding proposal, wherein each virtual IVD laboratory instrument 50 simulates the operational behavior of the respective IVD laboratory instrument 20.
Proposal 8: The method according to one of the proposals 6 or 7, wherein each virtual IVD laboratory instrument 50 simulates the communicational behavior of the respective IVD laboratory instrument 20.
Proposal 9: The method according to one of the preceding proposals, wherein the method comprises simulating samples processed in the IVD laboratory system 10 as virtual samples 52.
Proposal 10: The method according to the preceding proposal, wherein the method comprises displaying a visualization of the virtual IVD laboratory instrumentation 5 together with a visualization of virtual samples 52.
Proposal 11: The method according to the preceding proposal, wherein the virtual IVD laboratory instrumentation 5 is displayed as a schematic representation corresponding to the IVD laboratory instrumentation 2 to be simulated.
Proposal 12: The method according to the preceding proposal, wherein the virtual samples 52 each are displayed at a position in the schematic representation corresponding to a position of a corresponding sample to be simulated at the time.
Proposal 13: The method according to one of the preceding proposals, wherein the design of the virtual IVD laboratory instrumentation 5 is created using a design file.
Proposal 14: The method according to one of the preceding proposals, wherein the design of the virtual IVD laboratory instrumentation 5 is created using a list of IVD laboratory instruments 20 defined in the IVD laboratory control software module 3.
Proposal 15: The method according to one of the preceding proposals, wherein the design of the virtual IVD laboratory instrumentation 5 are created using a graphical user interface 9.
Proposal 16: The method according to one of the preceding proposals, wherein the method comprises using a graphical user interface 9 that comprises a schematic representation of a visualization of details of the design of the virtual IVD laboratory instrumentation 5; wherein this graphical user interface 9 may e.g. be the graphical user interface of proposal 15.
Proposal 17: The method according to one of the preceding proposals, wherein
   - simulating sample processing within the IVD laboratory instrumentation 2 and
   - operating the IVD laboratory control software module 3,
   are both performed in real-time.
Proposal 18: The method according to one of the preceding proposals, wherein the method comprises
   - simulating sample processing within the IVD laboratory instrumentation 2 in a simulation time;
   - operating the IVD laboratory control software module 3 in real-time;
   - comparing the time passed in the simulation time to the real-time;
   - and:
      ∘ increasing the speed of the simulation time if the simulation time is behind the real-time,
      ∘ decreasing the speed of the simulation time if the simulation time is ahead of the real-time, or
      ∘ maintaining the speed of the simulation time if the simulation time is at least quasi-identical to the real-time.
Proposal 19: The method according to one of the preceding proposals, wherein the IVD laboratory control software module 3 is connected to a data management system 6 for exchanging sample related information SRI with the IVD laboratory control software module 3, and wherein the method comprises
   - exchanging sample related information SRI between the IVD laboratory control software module 3 and the data management system 6.
Proposal 20: The method according to one of the preceding proposals, wherein the method comprises
   - simulating a virtual data management system 56 configured for exchanging sample related information SRI with the IVD laboratory control software module 3, and
   - exchanging sample related information SRI between the IVD laboratory control software module 3 and the virtual data management system 56.
Proposal 21: The method according to the preceding proposal, wherein the method comprises
   - receiving by the IVD laboratory control software module 3 a first data from the virtual data management system 56 or from the virtual IVD laboratory instrumentation 5, the first data comprising an indication that a sample has arrived at the IVD laboratory instrumentation 2, thereby receiving a virtual sample;
   - receiving by the IVD laboratory control software module 3 a second data from the virtual data management system 56, the second data comprising a test order for the sample, thereby receiving a test order for the virtual sample;
   - simulating sample processing of this virtual sample according to the test order using the virtual IVD laboratory instrumentation 5.
Proposal 22: A method for designing an IVD laboratory control software module 3 using the method for simulating according to one of proposals 1 to 21, the method for designing comprising the following steps in the following order:
   - STEP 0: providing a design of the IVD laboratory control software module 3;
   - STEP 1: performing the method for simulating;
   - STEP 2: changing one or more parameters of the design of the IVD laboratory control software module 3;
   - STEP 3: performing STEP 1; and
   - STEP 4: optionally repeating STEP 2 and STEP 3.
Proposal 23: A method for designing an IVD laboratory instrumentation 2 using the method according to one of proposals 1 to 21, the method for designing comprising the following steps in the following order:
   - STEP 0: providing a design of the IVD laboratory instrumentation 2;
   - STEP 1: performing the method for simulating;
   - STEP 2: changing one or more parameter of the design of the IVD laboratory instrumentation 2;
   - STEP 3: performing STEP 1; and
   - STEP 4: optionally repeating STEP 2 and STEP 3.
Proposal 24: A method for designing an IVD laboratory system 10 using the method according to one of proposals 1 to 21, the method for designing comprising the following steps in the following order:
   - STEP 0: providing a design of the IVD laboratory control software module 3 and a design of the IVD laboratory instrumentation 2;
   - STEP 1: performing the method for simulating;
   - STEP 2: changing one or more parameter of the design of the IVD laboratory control software module 3 and/or of the design of the IVD laboratory instrumentation 2;
   - STEP 3: performing STEP 1; and
   - STEP 4: optionally repeating STEP 2 and STEP 3.
Proposal 25: The method for designing according to one of proposals 22 to 24, wherein STEP 1 comprises benchmarking the performance of the IVD laboratory control software module 3 resp. the IVD laboratory instrumentation 2.
Proposal 26: A method for testing an IVD laboratory control software module 3 using the method for simulating according to one of proposals 1 to 21.
Proposal 27: A method for testing an IVD laboratory instrumentation 2 using the method according to one of proposals 1 to 21.
Proposal 28: A method for testing an IVD laboratory system 10 using the method for simulating according to one of proposals 1 to 21.
Proposal 29: A method for testing personnel operation of an IVD laboratory system 10 using the method for simulating according to one of proposals 1 to 21.
Proposal 30: A method for training personnel for working in an IVD laboratory system 10 using the method for simulating according to one of proposals 1 to 21.
Proposal 31: A method for designing a virtual IVD laboratory instrument 50 for simulating an IVD laboratory instrument 20 for a method for simulating according to one of proposals 1 to 21, the method comprising
   - recording and/or defining data files being received and/or being sent by the IVD laboratory instrument 20, and/or
   - recording and/or defining time gaps between the receiving and/or the sending of the data files by the IVD laboratory instrument 20;
   - using the recorded resp. defined data files and/or the recorded resp. defined time gaps for creating the content and/or for defining the sending timing of data files by the virtual IVD laboratory instrument 50.
Proposal 32: A system 1 for simulating an IVD laboratory system 10 for processing biological samples, the IVD laboratory system 10 comprising
   - an IVD laboratory instrumentation 2 comprising one or more IVD laboratory instruments 20 for processing biological samples and
   - an IVD laboratory control software module 3 for controlling sample processing within the IVD laboratory instrumentation 2 and exchanging sample processing data SPD between the IVD laboratory control software module 3 and the IVD laboratory instrumentation 2,
   wherein the IVD laboratory system 10 is designed for
   - sample processing within the IVD laboratory instrumentation 2 using sample processing data SPD provided by the IVD laboratory control software module 3;
   wherein the system 1 comprises
   - the IVD laboratory control software module 3, and
   - a simulation software module 4 comprising a virtual IVD laboratory instrumentation 5 for simulating sample processing within the IVD laboratory instrumentation 2;
   wherein the system 1 is configured for
   - operating the IVD laboratory control software module 3 as if it was operating within the IVD laboratory system 10, but wherein sample processing data SPD intended for being exchanged between the IVD laboratory control software module 3 and the IVD laboratory instrumentation 2 is instead exchanged between the IVD laboratory control software module 3 and the virtual IVD laboratory instrumentation 5;
   wherein the virtual IVD laboratory instrumentation 5 is configured for
   - simulating sample processing within the IVD laboratory instrumentation 2 using sample processing data SPD provided by the IVD laboratory control software module 3.
Proposal 33: The system 1 according to proposal 32, wherein the IVD laboratory control software module 3 comprises a first communication module 31 comprising at least one driver 31d for transforming a data format of a data file.
Proposal 34: The system 1 according to one of proposals 32 to 33, wherein the simulation software module 4 comprises a second communication module 51 comprising at least one driver 51d for transforming a data format of a data file.
Proposal 35: The system 1 according to proposal 34 as depending on proposal 33, wherein the first communication module 31 and the second communication module 51 each comprise at least one joint driver 31d. 51d.
Proposal 36: The system 1 according to proposal 34 as depending on proposal 33 or according to proposal 35, wherein the operation of the first communication modules 31 is mirrored by the second communication module 51.

### REFERENCE LIST

| | | | |
|---|---|---|---|
| 1 | system | 5 | virtual IVD laboratory |
| 10 | IVD laboratory system | | instrumentation |
| 2 | IVD laboratory instrumentation | 50 | virtual IVD laboratory instrument |
| 20 | IVD laboratory instrument | 50pre | virtual pre-analytical IVD |
| 20pre | pre-analytical IVD laboratory | | laboratory instrument |
| | instrument | 50ana | virtual analytical IVD |
| 20ana | analytical IVD laboratory | | laboratory instrument |
| | instrument | 50post | virtual post-analytical IVD |
| 20post | post-analytical IVD laboratory | | laboratory instrument |
| | instrument | 50trans | virtual transport IVD |
| 20trans | transport IVD laboratory | | laboratory instrument |
| | instrument | 51 | virtual communication module |
| 3 | IVD laboratory control software | 51d | driver |
| | module | 52 | virtual sample |
| 30 | control components | 53 | virtual sample container holder |
| 30w | workflow engine | 56 | virtual data management system |
| 30q | quality control module | 6 | data management unit |
| 30r | rule engine | 7 | communication network |
| 30s | sample tracking module | 9 | graphical user interface |
| 30p | patient data module | SPD | sample processing data |
| 31 | communication module | SRI | sample related information |
| 31d | driver | IC | internal communication |
| 32 | control unit | | |
| 4 | simulation software module | | |

## Claims

1. A method for simulating an in-vitro diagnostic IVD laboratory system (10) for processing biological samples, the IVD laboratory system (10) comprising
- an IVD laboratory instrumentation (2) comprising one or more IVD laboratory instruments (20) for processing samples,
- an IVD laboratory control software module (3) for controlling sample processing within the IVD laboratory instrumentation (2) and exchanging sample processing data (SPD) between the IVD laboratory control software module (3) and the IVD laboratory instrumentation (2),
wherein the IVD laboratory system (10) is designed for
- sample processing within the IVD laboratory instrumentation (2) using sample processing data (SPD) provided by the IVD laboratory control software module (3);
the method comprising the steps of:
- simulating sample processing within the IVD laboratory instrumentation (2) using a virtual IVD laboratory instrumentation (5) using sample processing data (SPD) provided by the IVD laboratory control software module (3),
- operating the IVD laboratory control software module (3) as if it was operating within the IVD laboratory system (10) but wherein sample processing data (SPD) intended for being exchanged between the IVD laboratory control software module (3) and the IVD laboratory instrumentation (2) is instead exchanged between the IVD laboratory control software module (3) and the virtual IVD laboratory instrumentation (5).

2. The method according to the preceding claim, wherein the method comprises
- receiving a virtual sample and a receiving a test order for the virtual sample;
- simulating receiving of the virtual sample by the virtual IVD laboratory instrumentation (5) resp. a virtual IVD laboratory instrument (50) thereof;
- sending a processing query for a virtual sample from the virtual IVD laboratory instrumentation (5) resp. a virtual IVD laboratory instrument (50) thereof to the IVD laboratory control software module (3);
- sending, e.g. after having received a processing query, a processing order for a virtual sample generated using a test order for this virtual sample from the IVD laboratory control software module (3) to the virtual IVD laboratory instrumentation (5) resp. a virtual IVD laboratory instrument (50) thereof;
- simulating sample processing in the form of sample testing, e.g. according to a test order the virtual IVD laboratory instrumentation (5) resp. a virtual IVD laboratory instrument (50) thereof, e.g. by sending a, e.g. created and/or received, test result from the virtual IVD laboratory instrumentation (5) resp. a virtual IVD laboratory instrument (50) thereof to the IVD laboratory control software module (3),
∘ e.g. wherein the test result is sent with a time gap from the start of the simulation of the sample testing, wherein the time gap is indicative of a time needed for this sample testing in an IVD laboratory instrumentation (2) resp. an IVD laboratory instrument (20) thereof;
- evaluating a received test result by the IVD laboratory control software module (3);
- sending a transport query for a virtual sample from the virtual IVD laboratory instrumentation (5) resp. a virtual IVD laboratory instrument (50) thereof to the IVD laboratory control software module (3);
- sending, e.g. after having received a transport query, a transport order for a virtual sample from the IVD laboratory control software module (3) to the virtual IVD laboratory instrumentation (5) resp. a virtual IVD laboratory instrument (50) thereof;
- simulating a transport of the virtual sample within the virtual IVD laboratory instrumentation (5), e.g. according to a transport order, and/or
- sending positioning information from the virtual IVD laboratory instrumentation (5) resp. a virtual IVD laboratory instrument (50) thereof to the IVD laboratory control software module (3), the positioning information concerning a current simulated position of a virtual sample (52) within the virtual IVD laboratory instrumentation (5) resp. a virtual IVD laboratory instrument (50) thereof.

3. The method according to one of the preceding claims, wherein the method comprises simulating sample processing by personnel within the IVD laboratory system (10).

4. The method according to one of the preceding claims, wherein the virtual IVD laboratory instrumentation (5) comprises exactly one virtual IVD laboratory instrument (50) for each IVD laboratory instrument (20) of the IVD laboratory system (10) to be simulated, wherein each virtual IVD laboratory instrument (50) simulates the communicational behavior of the respective IVD laboratory instrument (20).

5. The method according to one of the preceding claims, wherein the method comprises simulating samples processed in the IVD laboratory system (10) as virtual samples (52), wherein the method comprises displaying a visualization of the virtual IVD laboratory instrumentation (5) together with a visualization of virtual samples (52).

6. The method according to one of the preceding claims, wherein
- simulating sample processing within the IVD laboratory instrumentation (2) and
- operating the IVD laboratory control software module (3),
are both performed in real-time.

7. The method according to one of the preceding claims, wherein the method comprises
- simulating sample processing within the IVD laboratory instrumentation (2) in a simulation time;
- operating the IVD laboratory control software module (3) in real-time;
- comparing the time passed in the simulation time to the real-time;
- and:
∘ increasing the speed of the simulation time if the simulation time is behind the real-time,
∘ decreasing the speed of the simulation time if the simulation time is ahead of the real-time, or
∘ maintain the speed of the simulation time if the simulation time is at least quasi-identical to the real-time.

8. The method according to one of the preceding claims,
wherein the IVD laboratory control software module (3) is connected to a data management system (6) for exchanging sample related information (SRI) with the IVD laboratory control software module (3), and
wherein the method comprises
- exchanging sample related information (SRI) between the IVD laboratory control software module (3) and the data management system (6).

9. The method according to one of the preceding claims, wherein the method comprises
- simulating a virtual data management system (56) configured for exchanging sample related information (SRI) with the IVD laboratory control software module (3), and
- exchanging sample related information (SRI) between the IVD laboratory control software module (3) and the virtual data management system (56).

10. A method for designing an IVD laboratory control software module (3) and/or an IVD laboratory instrumentation (2) using the method for simulating according to one of claims 1 to 9, the method for designing comprising the following steps in the following order:
- STEP 0: providing a design of the IVD laboratory control software module (3) and/or an IVD laboratory instrumentation (2);
- STEP 1: performing the method for simulating;
- STEP 2: changing one or more parameters of the design of the IVD laboratory control software module (3) and/or an IVD laboratory instrumentation (2);
- STEP 3: performing STEP 1; and
- STEP 4: optionally repeating STEP 2 and STEP 3.

11. The method for designing according to claim 10, wherein STEP 1 comprises benchmarking the performance of the IVD laboratory control software module (3) and/or the IVD laboratory instrumentation (2).

12. A method for testing an IVD laboratory control software module (3) and/or an IVD laboratory instrumentation (2) using the method for simulating according to one of claims 1 to 9.

13. A method for testing personnel operation of an IVD laboratory system (10) and/or for training personnel for working in an IVD laboratory system (10) using the method for simulating according to one of claims 1 to 9.

14. A method for designing a virtual IVD laboratory instrument (50) for simulating an IVD laboratory instrument (20) for a method for simulating according to one of claims 1 to 9, the method comprising
- recording and/or defining data files being received and/or being sent by the IVD laboratory instrument (20), and/or
- recording and/or defining time gaps between the receiving and/or the sending of the data files by the IVD laboratory instrument (20);
- using the recorded resp. defined data files and/or the recorded resp. defined time gaps for creating the content and/or for defining the sending timing of data files by the virtual IVD laboratory instrument (50).

15. A system (1) for simulating an IVD laboratory system (10) for processing biological samples, the IVD laboratory system (10) comprising
- an IVD laboratory instrumentation (2) comprising one or more IVD laboratory instruments (20) for processing biological samples and
- an IVD laboratory control software module (3) for controlling sample processing within the IVD laboratory instrumentation (2) and exchanging sample processing data (SPD) between the IVD laboratory control software module (3) and the IVD laboratory instrumentation (2),
wherein the IVD laboratory system (10) is designed for
- sample processing within the IVD laboratory instrumentation (2) using sample processing data (SPD) provided by the IVD laboratory control software module (3);
wherein the system (1) comprises
- the IVD laboratory control software module (3), and
- a simulation software module (4) comprising a virtual IVD laboratory instrumentation (5) for simulating sample processing within the IVD laboratory instrumentation (2);
wherein the system (1) is configured for
- operating the IVD laboratory control software module (3) as if it was operating within the IVD laboratory system (10), but wherein sample processing data (SPD) intended for being exchanged between the IVD laboratory control software module (3) and the IVD laboratory instrumentation (2) is instead exchanged between the IVD laboratory control software module (3) and the virtual IVD laboratory instrumentation (5);
wherein the virtual IVD laboratory instrumentation (5) is configured for
- simulating sample processing within the IVD laboratory instrumentation (2) using sample processing data (SPD) provided by the IVD laboratory control software module (3).
